# EUROPÄISCHE PATENTSCHRIFT

(11) **EP 2 726 120 B1**
(45) Veröffentlichungstag und Bekanntmachung des Hinweises auf die Patenterteilung: **01.09.2021**
(21) Anmeldenummer: 12729887.5
(22) Anmeldetag: 27.06.2012
(51) Int. Cl.: A61M 1/16, A61M 1/28

(54) **PERITONEALDIALYSEMASCHINE**
PERITONEAL DIALYSIS MACHINE
MACHINE DE DIALYSE PÉRITONÉALE

(30) Priorität: 29.06.2011 DE 102011105916; 29.06.2011 US 201161502467 P
(43) Veröffentlichungstag der Anmeldung: 07.05.2014
(73) Patentinhaber: Fresenius Medical Care Deutschland GmbH, 61352 Bad Homburg (DE)
(72) Erfinder: GRIESMANN, Erik, 97422 Schweinfurt (DE); HEDMANN, Frank, 97332 Volkach (DE); WICH-HEITER, Joachim, 97273 Kürnach (DE)
(74) Vertreter: Laufhütte, Dieter
(86) Internationale Anmeldenummer: PCT/EP2012/002701
(87) Internationale Veröffentlichungsnummer: WO 2013/000569

(56) Entgegenhaltungen:
- WO-A1-99/51287
- WO-A1-2007/091217
- WO-A1-2010/108955
- DE-A1- 1 964 733
- DE-U1- 9 203 785
- US-A- 5 634 896
- US-A1- 2009 294 339
- US-A1- 2010 137 782
- US-A1- 2010 191 180

## Beschreibung

Die vorliegende Erfindung betrifft eine Dialysemaschine sowie ein Verfahren zum Betrieb einer Dialysemaschine.

Bei der Durchführung einer Peritonealdialysebehandlung wird eingangs der Behandlung eine geplante Behandlungsdauer vorgegeben. Die geplante Behandlungsdauer ergibt sich aus den einzelnen Einfüll-, Verweil- und Auslaufphasen eines jeden Zyklus einer Peritonealdialysebehandlung.

Ferner wird für die Durchführung einer Peritonealdialysebehandlung dem Patienten ein Behandlungsvolumen verschrieben. Hieraus ergibt sich sodann, welche Beutel an die Dialysemaschine anzuschließen sind. Die Dialyselösung kann sich aus dem Beutelinhalt mehrerer, in den jeweiligen Beuteln enthaltener Lösungen zusammensetzen. Die Beutelauswahl in Bezug auf die Beutelgröße erfolgt durch den Patienten selbst, wobei der Patient nur die jeweiligen Beutel und ein ausreichendes Nominalvolumen anschließen muss.

Zu beachten ist, dass die Dialysemaschine einen Eigenverbrauch an Lösung hat, der beispielsweise durch Kalibrierungs- und Spülvorgänge der Dialysemaschine bedingt ist, wobei diese hierfür verbrauchte Lösung danach verworfen und nicht für Behandlungszwecke verwendet wird. Somit ist bei jeder Behandlung ein Verwurf an Lösung vorhanden.

Üblicherweise wird das zur Verfügung stehende Mindestbehandlungsvolumen an Dialysat zyklisch in mehreren Teilvolumina, die auch als Einlaufvolumen bezeichnet werden, in das Peritoneum des Patienten infundiert und verbleibt dort für eine vordefinierte Verweilzeit, auch Verweilphase oder Dwellzeit/-phase genannt.

Als nachteilig werden beispielsweise Verlängerungen der Behandlungsdauer wahrgenommen, ebenso ist es nachteilig, wenn das zur Verfügung stehende Dialysatvolumen nicht vollständig ausgenutzt werden kann, weil z. B. das verbleibende Restvolumen nicht für eine adäquate Behandlung ausreicht und deshalb nicht infundiert wird, sondern verworfen wird.

Die tatsächliche Behandlungsdauer kann durch mehrere Faktoren beeinflusst werden, nämlich insbesondere durch die tatsächlich erreichten Flussraten bei Ein- und Ausläufen des Dialysats, das tatsächlich verabreichte Volumen für Einläufe und das vom Patienten generierte Ultrafiltrationsvolumen, das bei Ausläufen zusätzlich entnommen wird.

Des Weiteren kann es zu Veränderungen hinsichtlich der benötigten Behandlungsdauer kommen, nämlich z. B. in Folge einer Änderung des Behandlungsverlaufes durch den Patienten (z. B. manueller Auslauf oder Überspringen von Behandlungszyklen), einer Änderung von Parametern der Behandlungsverschreibung während der Behandlung (Ändern von Volumina für nachfolgende Einläufe, Ändern von Verweilzeiten) oder durch eine Unterbrechung bzw. Pause der Behandlung durch den Patienten.

Auch das zur Verfügung stehende Beutelvolumen ändert sich im Laufe der Behandlung insofern, dass die betreffenden Lösungen bereits dem Patienten verabreicht wurden. Die Änderung des Beutelvolumens kann weiterhin dadurch begründet sein, dass die Lösung für interne Prozesse benötigt wurde (z. B. zum Justieren der Pumpe, Priming usw.) oder dass die Lösung auf Grund von Alarmen verworfen wird (z. B. zu heiße Flüssigkeit). Auch ist möglich, dass die für die Behandlung benötigte Einlauflösung variiert, etwa durch eine nutzerseitige Änderung des Behandlungsverlaufs (z. B. manueller Auslauf oder Überspringen eines Zyklus) oder durch ein Ändern von Parametern der Behandlungsverschreibung während der Behandlung (z. B. ein Ändern von Volumina für nachfolgende Einläufe).

Aus der WO 2009/149002 A2 ist eine Peritonealdialysemaschine bekannt, die über eine Eingabe die Veränderung von angezeigten Behandlungsparametern ermöglicht.

Die WO 1999/51287 A1 beschreibt eine Peritonealdialysemaschine, die im TPD-Modus (Tidal Peritoneal Dialysis Modus) betreibbar ist.

Die WO 2007/091217 A1 offenbart eine Peritonealdialysemaschine, die eine Veränderung der Verweilzeit des Dialysates im Patienten ermöglicht.

Die WO 2005/035023 A1 betrifft eine Peritonealdialysemaschine, bei der mittels eines Sensors der Dialysatabfluss aus dem Peritoneum des Patienten überwacht wird. Der Dialysatabfluss wird dabei unterbrochen, wenn ein Schwellwert erreicht wird.

Aus der WO 2006/034178 A2 offenbart eine Peritonealdialysemaschine, bei der eine Ermittlung des noch nicht infundierten Dialysates erfolgt.

Die JP 2009-011667 A beschreibt eine Peritonealdialysemaschine, die den Bedarf an Dialyseflüssigkeit berechnet.

Die US 2010/0191180 A1 betrifft eine Peritonealdialysemaschine, die Anpassungen von Behandlungsparametern ermöglicht.

Die WO 2010/108955 A1 offenbart eine Dialysevorrichtung, die eine automatisierte Peritonealdialysemaschine umfasst, mit einer Verschreibungsschnittstelle und einem Programmiermittel, das konfiguriert ist, um über die Verschreibungsschnittstelle die Eingabe eines Satzes von therapeutischen Zielen und eines Behandlungsplans zu ermöglichen, und einem Wiederbewertungsmittel, das konfiguriert ist, als Funktion der therapeutischen Ziele und des Behandlungsplans, mindestens eine Behandlungsoption vorzuschlagen, die einen Satz von Behandlungseinstellungen zum Erreichen des Satzes von therapeutischen Zielen umfasst. Der Satz von Einstellungen umfasst eine Anzahl von individuellen Einstellungen, wobei die individuelle Einstellung die Behandlungssitzungsdauer, das Flüssigkeitsvolumen pro Austausch, die Verweildauer, oder das Gesamtvolumen der Dialyseflüssigkeit während einer Behandlungssitzung sein kann. Die Dialysevorrichtung dieser Druckschrift kann eine Patientenschnittstelle und eine Änderungseinrichtung umfassen, die konfiguriert ist, um über die Patientenschnittstelle eine Änderung mindestens einer der Einstellungen zu ermöglichen, wobei das Wiederbewertungsmittel konfiguriert ist, als Funktion der geänderten Einstellung, die mindestens eine Behandlungsoption vorzuschlagen. Der Patient kann Einstellungen während einer laufenden Behandlungssitzung ändern. Diese Dialysevorrichtung kann ein zustandsbestimmendes Mittel umfassen, das konfiguriert ist, klinische oder therapeutische Parameter zu messen, wobei das Wiederbewertungsmittel konfiguriert ist, als Funktion der gemessenen Parameter während einer laufenden Behandlungssitzung oder gemessener Parameter mindestens einer vorhergehenden Behandlungssitzung, die mindestens eine Behandlungsoption vorzuschlagen. Mittels dieser Dialysevorrichtung ist es möglich, eine optimierte Nutzung der verfügbaren Flüssigkeiten in Abhängigkeit von der Behandlungssitzungsdauer anzustreben. Dabei ist möglich, die Dauer der Verweilzeit zu verkürzen, so dass sichergestellt ist, dass die Flüssigkeit aus allen Beuteln während der verfügbaren Zeit für die Behandlungssitzung verwendet wird, während die beabsichtigte Anzahl von Fluidaustauschen eingehalten wird. Der Patient kann die Dauer der Behandlung von einem Tag zum anderen ändern. Beispielsweise kann die Vorrichtung als Funktion der vorhergehenden Sitzungen die Programmierung einer Behandlungssitzungsdauer von 8 bis 12 Stunden in normaler Zeit, oder von 10 bis 14 Stunden, wenn ein kumulatives therapeutisches Defizit aufgrund zu kurzer Behandlungssitzungen in den letzten Tagen eingetragen werden, oder von mindestens 14 Stunden, wenn dieses Defizit als zu groß betrachtet wird, ermöglichen oder sogar eine zusätzliche Tagesbehandlung vorschlagen.

Bei der Peritonealdialysevorrichtung der US 2010/0137782 A1 werden vorgegebenen Behandlungsparameter vorgegeben, insbesondere ein erster Parameter, der ein gewünschtes im abdominalen Höhlraum des Patienten nach jeder Füllperiode F der Behandlung zu enthaltendes Zielvolumen Vt betrifft, ein zweiter Parameter, der ein gesamtes während der Behandlung zu gebrauchendes Volumen an Dialysatfluid betrifft, und ein dritter Parameter, der eine gesamte Behandlungszeitdauer betrifft. Ein Prozessor der Vorrichtung ist konfiguriert, anhand dieser Parameter ein Füllvolumen an Dialysatfluid und eine Füll-/Verweilzeit, umfassend die Zeit der Füllperiode und der Verweilperiode, zu berechnen. Nach dem ersten Zyklus ist der Prozessor konfiguriert, unter Berücksichtigung der Füll-/Verweilzeit und des Füllvolumens des vorhergehenden Zyklus die Parameter anzupassen. Der Prozessor ist auch konfiguriert, auf Basis der angepassten Parameter ein neues Füllvolumen VF und eine neue Füll-/Verweilzeit zu berechnen.

Bei der Peritonealdialysebehandlung der US 2009/0294339 A1 wird der Patient zur Einhaltung eines vorgegebenen Behandlungsendzeitpunktes von der Dialysemaschine benachrichtigt, dass die Maschine die Therapie auf Basis des Zeitpunkts, wo der Patient die Therapie tatsächlich beginnt, automatisch anpasst. Eine Alarmlage erfolgt, wenn die gesamte Behandlungsdauer nicht ausreichende Verweilzeit für eine effektive Behandlung ermöglicht. Abhängig vom Zeitpunkt, wo der Patient die Therapie tatsächlich beginnt, passt die Maschine die Verweilzeiten auf Basis berechneter oder abgeschätzter Füll- und Entleerungszeiten an.

Weitere Peritonealdialysemaschinen sind aus der DE 92 03 785 U1 und der DE 19 64 733 A1 bekannt.

Es ist daher die Aufgabe der vorliegenden Erfindung, eine Dialysemaschine der eingangs genannten Art in vorteilhafter Weise weiterzubilden, insbesondere dahingehend, dass die geplante Behandlungsdauer im Wesentlichen eingehalten und/oder das zur Verfügung stehende Dialysatvolumen optimal genutzt werden kann.

Diese Aufgabe wird erfindungsgemäß durch eine Dialysemaschine mit den Merkmalen des Anspruchs 1 gelöst. Danach ist vorgesehen, dass eine Dialysemaschine derart ausgeführt wird, dass mittels der Dialysemaschine gemäß vorgegebenen und/oder eingebbaren Behandlungsparametern eine Peritonealdialysebehandlung durchführbar ist und wobei die Dialysemaschine wenigstens ein Einstellmittel aufweist, mittels dessen wenigstens ein Behandlungsparameter während des Behandlungsbetriebes der Dialysemaschine einstellbar ist.

Insbesondere die Teilvolumina bzw. Einlaufvolumina, die Anzahl der Behandlungszyklen, das zu verwendende Dialysat und die Dwellzeit werden als Behandlungsparameter vor der Durchführung der Behandlung festgelegt und als Behandlungsplan in der Dialysemaschine hinterlegt. Vor Beginn der Behandlung ist das zur Verfügung stehende Mindestbeutelvolumen aller angeschlossenen Einlauflösungen bekannt, da diese vor der Konnektion z. B. via Barcode identifiziert werden. Durch die Identifizierung stehen die Eigenschaften der Lösungen als Information bzw. weitere Behandlungsparameter maschinenseitig zur Verfügung. Insbesondere handelt es sich um Informationen hinsichtlich Lösungsart, Beutelgröße, Mindestüberfüllungsvolumen, nominales Füllvolumen, Glucosekonzentration, Calciumkonzentration, Beutelart wie z. B. Doppelkammerbeutel oder Einkammerbeutel. Das Mindestüberfüllungsvolumen ist dabei das vom Hersteller garantierte Lösungsvolumen, welches zusätzlich zum nominalen Füllvolumen in einem Lösungsbeutel vorhanden ist. Das Einlaufvolumen ist dabei das Volumen an Dialysat, das bei einem Behandlungszyklus in den Patienten infundiert wird bzw. für die Infundierung in den Patienten vorgesehen bzw. bestimmt ist.

Es ergibt sich insbesondere der Vorteil und es ist besonders vorteilhaft möglich, dass die Einstellung des wenigstens einen Behandlungsparameters automatisch angepasst werden kann.

Der oder die Behandlungsparameter ist bzw. sind die Verweilzeit und/oder das Einlaufvolumen, die während der Behandlung geändert werden können. Die Verweilzeit (dwell time) ist dabei die Verweilzeit des Dialysates im Peritoneum des zu behandelnden Patienten.

Es ist somit vorteilhaft möglich, die Verweilzeit des Dialysates im Peritoneum und das Dialysatvolumen für die Befüllung des Peritoneums während der einzelnen Zyklen einer Dialysebehandlung variieren zu können. Insbesondere ist möglich, das Einlaufvolumen und die Verweilzeit, auch Dwellzeit genannt, unter bestimmten Voraussetzungen innerhalb eines vorgegebenen Toleranzrahmens kürzen zu können. Damit soll z. B. bei der Kürzung der Einlaufvolumina während der Zyklen eine möglichst optimale Ausnutzung der Gesamtdialysemenge erreicht werden, sowie durch die Kürzung der Verweilzeit die Behandlung nach der Dauer der verschriebenen bzw. geplanten Behandlungszeit auch abgeschlossen werden können. Trotz dieser Maßnahmen wird dabei sichergestellt, dass aufgrund der Einstellungsmöglichkeit der Behandlungsparameter Verweilzeit und/oder Einlaufvolumen das Behandlungsziel erreicht werden kann.

Insbesondere kann nun erreicht werden, dass die an die Dialysemaschine angeschlossenen Beutel so gut wie möglich ausgenutzt werden können.

Die erfindungsgemäße Dialysemaschine weist ein Planungsmittel zur Planung eines Behandlungsverlaufes und ein Überwachungsmittel zur Überwachung des Behandlungsverlaufes auf, wobei mittels des Einstellmittels abgleichbar ist, ob ein tatsächlicher Behandlungsverlauf mit einem geplanten Behandlungsverlauf übereinstimmt oder abweicht. Der geplante Behandlungsverlauf kann beispielsweise über eine Patientenkarte an das Planungsmittel übergeben werden. Auch ist denkbar, dass eine derartige Patientenkarte Bestandteil des Planungsmittels ist.

In einer ersten Ausgestaltung der vorliegenden Erfindung ist mittels des Planungsmittels wenigstens die geplante Behandlungsdauer vorgebbar und mittels des Überwachungsmittels wenigstens die voraussichtliche Behandlungsdauer anhand des bisherigen Behandlungsfortschritts errechenbar und/oder abschätzbar. Dabei sind während der gesamten Behandlungsdauer mehrere Verweilzeiten vorgesehen, deren Länge im Planungsmittel vorgebbar und/oder vorgegeben und eingespeichert ist bzw. sind, wobei mittels des Einstellmittels anhand der Errechnung und/oder Abschätzung des Überwachungsmittels der voraussichtlichen Behandlungsdauer eine Korrektur wenigstens einer Verweilzeit durchführbar ist, so dass die geplante Behandlungsdauer eingehalten werden kann.

Die dynamische Anpassung der Verweilzeiten vor jeder Verweilphase bzw. Dwellphase führt dazu, dass die Verweilphasen relativ gleichmäßig gekürzt werden. Da die Generierung von Ultrafiltrationsvolumen bzw. Stoffabtransport mit der Dauer der Dwellphase stark abnimmt, kann trotz Kürzung der Verweilphase eine adäquate Behandlung durchgeführt werden, nämlich insbesondere das Behandlungsziel erreicht werden. Diese dynamische Anpassung kann noch vorteilhafterweise mit einer Ausgestaltung der Dialysemaschine kombiniert werden, bei der vor dem letzten Behandlungszyklus beurteilbar ist, ob eine Kürzung der Verweilphase durchgeführt werden soll. Falls die Verweilphasendauer z. B. ca. 5 Minuten unterschreitet, wird der Behandlungszyklus nicht durchgeführt bzw. nicht seitens der Maschine gestartet. Nunmehr erfolgt diese Beurteilung aus Effizienz- und Komfortgründen. Die Ineffizienz der bloßen Überprüfung der letzten Verweilphasendauer wird nunmehr vorteilhaft dadurch begegnet, dass Behandlungsverzögerungen bereits über die gesamte oder zumindest über einen Teil der Behandlungsdauer kompensiert wurden. Besonders vorteilhaft ist nunmehr, dass ein ineffizienter letzter Behandlungszyklus wegen vergleichsweise geringer Verweildauer vermieden wird. Eine weitere Effizienzsteigerung ergibt sich dadurch, dass nun nicht mehr der Fall eintreten kann, dass aufgrund zu kurzer Verweildauer der letzte Behandlungszyklus wegfällt und hierdurch wertvolle Dialyselösung ungenutzt verworfen wird.

Es ist möglich, dass das Einstellmittel ein Begrenzungsmittel aufweist, mittels dessen ein prozentualer Faktor vorgebbar ist, der die maximal mögliche Korrektur der Verweilzeit begrenzt, wobei der prozentuale Faktor vorzugsweise 10 % der geplanten Verweilzeit beträgt.

Denkbar ist ferner, dass mittels des Einstellmittels die Zeitdifferenz von geplanter zu tatsächlicher Behandlungsdauer errechenbar ist und dass die errechnete Zeitdifferenz von der bislang vorgegebenen Verweilzeit mittels des Einstellmittels abziehbar und eine dementsprechend um die errechnete Zeitdifferenz korrigierte Verweilzeit einstellbar ist.

In einer zweiten Ausgestaltung der vorliegenden Erfindung ist mittels des Planungsmittels wenigstens ein geplantes Einlaufvolumen, insbesondere alle noch geplanten Einlaufvolumina, an Dialysat vorgebbar und mittels des Überwachungsmittels wenigstens das momentan verfügbare Volumen an Dialysat errechenbar und/oder abschätzbar, wobei mittels des Einstellmittels anhand der Errechnung und/oder Abschätzung des Überwachungsmittels des momentan verfügbaren Volumens an Dialysat eine Korrektur wenigstens eines geplanten Einlaufvolumens und/oder aller noch geplanten Einlaufvolumina durchführbar ist.

Es kann sich beispielsweise die Situation ergeben, dass das noch zur Verfügung stehende Volumen an Dialysat nicht mehr für sämtliche noch geplanten Einlaufvolumina ausreicht. Bei einem Festhalten an den geplanten Einlaufvolumina könnte sich somit beispielsweise ergeben, dass ein Behandlungszyklus aufgrund fehlenden Volumens an Dialysat entfallen müsste. In einem derartigen Fall ist es jedoch vorzuziehen, mehrere, insbesondere sämtliche geplanten Einlaufvolumina zu kürzen, um sämtliche geplanten Behandlungszyklen mit jetzt verminderten Einlaufvolumina beibehalten zu können. Denn aufgrund des Konzentrationsunterschiedes erfolgt der Übertritt der mittels der Dialysebehandlung zu entfernenden Stoffe bzw. Substanzen in das Dialysat wesentlich effektiver zu Beginn eines Behandlungszyklus, so dass es vorteilhafter ist, alle Behandlungszyklen beizubehalten, um diesen Effekt des verbesserten Stoffübertritts in das frische Dialysat zu nutzen und dafür ggf. das jeweilige Einlaufvolumen eines Behandlungszyklus zu kürzen.

Die Dialysemaschine ist gemäß der zweiten Ausgestaltung hierbei so ausgestaltet, dass vor Beginn der Behandlung ein zur Verfügung stehendes Mindestbeutelvolumen aller angeschlossenen Einlauflösungen maschinenseitig bekannt ist, indem diese identifiziert werden, wobei sich das Mindestbeutelvolumen aus einem nominalen Beutelfüllvolumen und einem garantierten Überfüllungsvolumen ergibt, die ebenfalls vor Beginn der Behandlung maschinenseitig bekannt sind, wobei die Dialysemaschine hierdurch so ausgestaltet ist, dass das Überfüllungsvolumen für den Eigenverbrauch der Maschine verwendet wird und eine Anpassung erfolgt, wenn das Überfüllungsvolumen für den Eigenverbrauch der Maschine nicht ausreicht.

Da die Einlaufvolumina vor jedem Einlauf vorteilhafterweise dynamisch mittels des Einstellmittels anpassbar sind, kann die zur Verfügung stehende Lösungsmenge, die der Dialysemaschine wie vorstehend bereits beschrieben hinsichtlich der relevanten Einzelheiten und damit auch hinsichtlich des Mindestbehandlungsvolumens bekannt ist, optimal aufgebraucht werden. Es ist nicht mehr notwendig, ein Sicherheitsvolumen für die Behandlung zu veranschlagen. Dies erhöht die Transparenz von Verschreibung zu benötigten Beuteln. So können nunmehr für eine 15 I Behandlung z. B. entweder drei Beutel je 5 I oder fünf Beutel je 3 I verwendet werden, obwohl das garantierte Mindestvolumen, welches sich aus dem nominalen Beutelfüllvolumen und dem garantierten Überfüllungsvolumen ergibt, der beiden Varianten unterschiedlich ist. Ein weiterer Vorteil ergibt sich ferner dadurch, dass im Gegensatz zu einer Kürzung eines Einlaufes am Ende der Behandlung (durch Alarm) die Einläufe gleichmäßig gekürzt werden. Dies ermöglicht, wie vorstehend bereits im Detail erläutert, eine adäquatere Behandlung für den Patienten, also ein besseres Erreichen des Behandlungsziels. Des Weiteren ist es nicht mehr erforderlich, z. B. bei Nachtbehandlungen und schlafendem Patienten diesen durch Alarm zu wecken, um den Einlauf aufgrund fehlender Lösung abzubrechen.

Darüber hinaus ist möglich, dass mittels des Einstellmittels anhand der Errechnung und/oder Abschätzung des Überwachungsmittels des momentan verfügbaren Volumens an Dialysat eine Korrektur wenigstens eines geplanten Einlaufvolumens und/oder aller noch geplanten Einlaufvolumina durchführbar ist.

Es ist denkbar, dass das Einstellmittel ein Begrenzungsmittel aufweist, mittels dessen ein prozentualer Faktor vorgebbar ist, der die maximal mögliche Korrektur des geplanten Einlaufvolumens begrenzt, wobei der prozentuale Faktor vorzugsweise 10 % des geplanten Einlaufvolumens eines jeden Zyklus beträgt.

Des Weiteren kann vorgesehen sein, dass mittels des Einstellmittels die Volumendifferenz von geplantem zu tatsächlichem Einlaufvolumen bzw. von den geplanten zu den tatsächlichen Einlaufvolumina errechenbar ist und dass das errechnete Einlaufvolumen bzw. die errechneten Einlaufvolumina von dem bislang vorgegebenen Einlaufvolumen bzw. von den bislang vorgegebenen Einlaufvolumina mittels des Einstellmittels abziehbar und dementsprechend um die errechnete Volumendifferenz ein korrigiertes Einlaufvolumen bzw. korrigierte Einlaufvolumina einstellbar ist bzw. sind.

Bei einem Verfahren zum Betrieb einer erfindungsgemäßen Dialysemaschine kann vorgesehen sein, dass gemäß vorgegebenen und/oder eingebbaren Behandlungsparametern eine Dialysebehandlung, insbesondere eine Peritonealdialysebehandlung, durchgeführt wird und wobei maschinenseitig wenigstens ein Behandlungsparameter dynamisch und/oder während des Behandlungsbetriebes der Dialysemaschine eingestellt werden kann und/oder wird.

Die dynamische und insbesondere automatische Einstellung ermöglicht es, dass die Behandlung innerhalb eines Toleranzbandes durchgeführt werden kann. Dieses Toleranzband berücksichtigt dabei sowohl den zeitabhängigen (horizontalen) als auch den volumenabhängigen (vertikalen) Aspekt. Durch die Festlegung der Faktoren für das Zeit- und Volumenmanagement kann das Toleranzband in horizontaler und vertikaler Richtung angepasst werden. Somit wird eine vorgegebene Behandlung vorteilhafterweise innerhalb des vorgegebenen Toleranzbandes durchgeführt.

Weitere Einzelheiten und Vorteile der vorliegenden Erfindung sollen nun anhand eines in der Zeichnung dargestellten Ausführungsbeispiels näher erläutert werden.

Dabei zeigen:
- Fig. 1: drei Diagramme, welche typische Verläufe einer automatischen Peritonealdialysebehandlung zeigen,
- Fig. 2: eine Prinzipdarstellung eines Peritonealdialysesystems,
- Fig. 3: eine Prinzipdarstellung der Aufteilung des Peritonealdialysesystems in eine Dialysemaschine und ein Fluidsystem,
- Fig. 4: ein erstes Ausführungsbeispiel einer Kassette,
- Fig. 5: ein zweites Ausführungsbeispiel einer Kassette,
- Fig. 6: eine perspektivische Ansicht eines ersten Ausführungsbeispiels einer Dialysemaschine,
- Fig. 7: ein Flussdiagramm eines ersten Ausführungsbeispiels eines Peritonealdialysesystems,
- Fig. 8: eine perspektivische Ansicht eines zweiten Ausführungsbeispiels einer Dialysemaschine,
- Fig. 9: ein Flussdiagramm eines zweiten Ausführungsbeispiels eines Peritonealdialysesystems,
- Fig. 10: das Ankoppeln der Kassette beim zweiten Ausführungsbeispiels eines Peritonealdialysesystems,
- Fig. 11: ein erstes Ausführungsbeispiel eines Pumpaktors,
- Fig. 12: das Ankoppeln eines Pumpbereichs der Kassette an einen Pumpaktor, und
- Fig. 13: eine Prinzipdarstellung des Aufbaus eines Ausführungsbeispiels einer Steuerung.

Im Folgenden soll die Funktion einer Dialysemaschine, bei welcher die vorliegende Erfindung zum Einsatz kommt, zunächst allgemein beschrieben werden. Bei der Dialysemaschine handelt es sich dabei im Ausführungsbeispiel um eine Peritonealdialysemaschine. Die unten beschriebenen Komponenten können jedoch in gleicher oder ähnlicher Weise auch für eine Hämodialysemaschine eingesetzt werden.

Die Peritonealdialyse ist eine Variante der künstlichen Blutwäsche, bei welcher das gut durchblutete Bauchfell (Peritoneum) des Patienten als körpereigene Filtermembran verwendet wird. Hierfür wird über einen Katheter Dialysat in die Bauchhöhle eingeführt. Nach dem Prinzip der Osmose diffundieren nun Harnbestandteile des Blutes durch das Bauchfell in das in der Bauchhöhle befindliche Dialysat. Nach einer gewissen Verweilzeit wird das Dialysat mit den Harnbestandteilen wieder aus der Bauchhöhle ausgelassen.

Bei der automatischen Peritonealdialyse steuert und überwacht eine Dialysemaschine die Einführung des frischen Dialysats in die Bauchhöhle und das Auslassen des verbrauchten Dialysats. Eine solche Dialysemaschine, auch Cycler genannt, befüllt und entleert die Bauchhöhle dabei üblicherweise mehrmals über Nacht, d.h. während der Patient schläft.

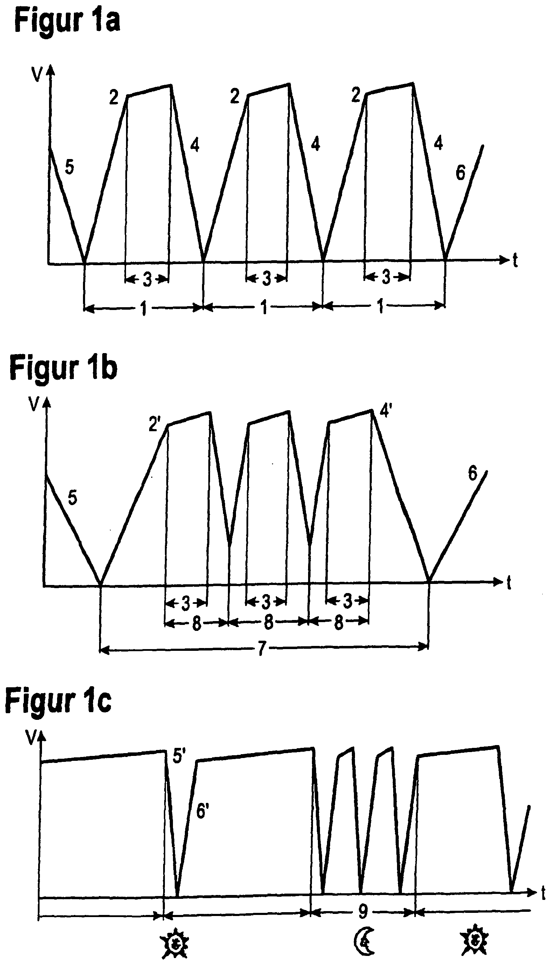

In Figuren 1a bis 1c sind drei unterschiedliche Verfahrensabläufe gezeigt, wie sie von einer Dialysemaschine durchgeführt werden. Einer oder mehrerer dieser Verfahrensabläufe ist dabei üblicherweise in der Steuerung der Dialysemaschine hinterlegt. Dabei ist es üblicherweise möglich, die abgespeicherten Verfahrensabläufe an den Patienten anzupassen.

Bei den Figuren 1a bis 1c ist jeweils die in der Bauchhöhle des Patienten befindliche Dialysatmenge V über die Zeit t aufgezeichnet. Figur 1a zeigt dabei den Verlauf einer normalen automatischen Peritonealdialysebehandlung über Nacht. Zu Beginn der Behandlung erfolgt dabei zunächst ein initialer Auslauf 5, durch welchen Dialysat, das über den Tag in der Bauchhöhle des Patienten belassen worden war, entnommen wird. Daraufhin folgen mehrere Behandlungszyklen 1, in Figur 1a drei aufeinander folgende Behandlungszyklen 1. Jeder Behandlungszyklus besteht dabei aus einer Einlaufphase 2, einer Verweilphase 3 und einer Auslaufphase 4. Während der Einlaufphase 2 wird dabei ein gewisses Volumen an frischer Dialyseflüssigkeit in die Bauchhöhle des Patienten eingelassen. Die maximal zulässige Dialysatmenge beträgt dabei je nach Patienten zwischen ca. 1,5 und 3 I. Das frische Dialysat verbleibt nun über eine gewisse Verweilzeit 3 in der Bauchhöhle. Typischerweise dauert die Verweilphase dabei einige Stunden. Daraufhin wird das nun verbrauchte Dialysat in der Auslaufphase 4 wieder aus der Bauchhöhle herausgelassen. Daraufhin startet ein neuer Behandlungszyklus. Die Behandlung wird mit einem letzten Einlauf 6 abgeschlossen, durch welchen eine gewisse Menge an frischem Dialysat in die Bauchhöhle des Patienten eingeführt wird. Diese verbleibt dann über den Tag in der Bauchhöhle des Patienten.

Die einzelnen Behandlungszyklen 1, welche über Nacht erfolgen, werden dabei automatisch von der Steuerung der Dialysemaschine angesteuert. Der initiale Auslauf und der letzte Einlauf können ebenfalls automatisch von der Dialysemaschine angesteuert werden. Alternativ werden diese manuell von einer Bedienperson oder vom Patienten aktiviert.

In Figur 1b ist eine sogenannte Tidalbehandlung gezeigt. Auch diese beginnt mit einem initialen Auslauf 5 und endet mit einem letzten Einlauf 6. Weiterhin ist ein Basiszyklus 7 vorgesehen, welcher sich in mehrere Tidalzyklen 8 aufteilt. Dabei ist zunächst eine Basiseinlaufphase 2' vorgesehen. Nach der Verweilphase 3 wird jedoch nicht mehr das komplette Dialysatvolumen aus der Bauchhöhle entnommen, sondern nur eine gewisse Teilmenge des in der Bauchhöhle befindlichen Dialysats. Dieses wird dann durch ein entsprechendes Volumen an frischem Dialysat ersetzt. Nach einem erneuten Verweilzyklus kann eine weitere Tidalentnahme erfolgen, bei welcher nicht das gesamte im Bauchraum befindliche Dialysat entfernt wird. Zum Ende des Basiszyklus 7 erfolgt eine Basisauslaufphase 4', bei welcher nun das gesamte Dialysat entnommen wird. In Figur 1b ist dabei lediglich ein Basiszyklus 1 dargestellt. Alternativ können jedoch auch mehrere Basiszyklen vorgesehen sein.

In Figur 1c ist der Verlauf einer Peritonealdialysebehandlung mit einer sogenannten PD-Plus-Behandlung gezeigt. Dabei erfolgt während der Nacht 9 eine übliche Peritonealdialysebehandlung, welche z. B. gemäß den Figuren 1a oder 1b durchgeführt werden kann. Weiterhin ist jedoch während des Tages eine zusätzliche PD-Plus-Behandlung vorgesehen, bei welcher in einer Auslaufphase 5' das verbrauchte Dialysat entnommen und in einer Einlaufphase 6' durch frisches Dialysat ersetzt wird. Bei der PD-Plus-Behandlung wird damit eine normale nächtliche Peritonealdialysebehandlung mit einem oder mehreren zusätzlichen Behandlungszyklen während des Tages kombiniert. Der Verlauf der nächtlichen Behandlung wird dabei wie üblich automatisch durch die Dialysemaschine durchgeführt. Die Behandlungszyklen während des Tages werden ebenfalls über die Maschine durchgeführt und überwacht.

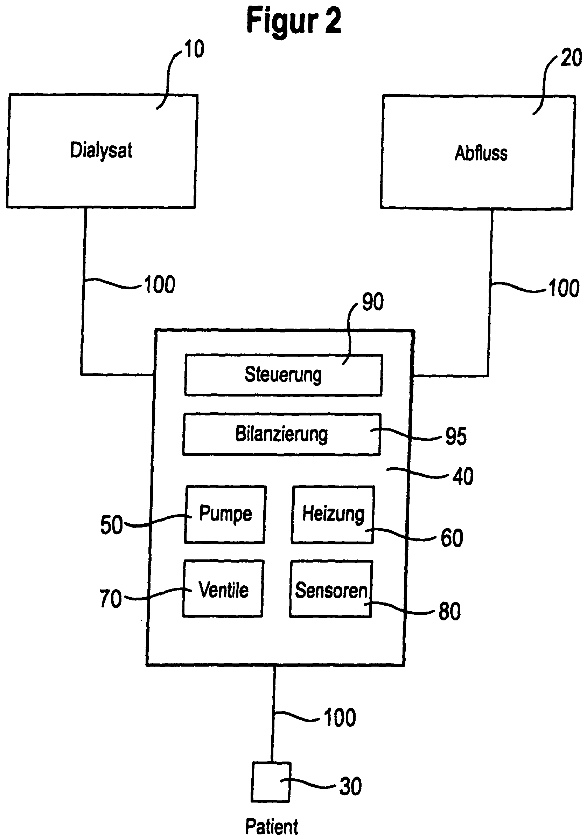

In Figur 2 ist nun der Aufbau eines typischen Peritonealdialysesystems schematisch dargestellt. Das Peritonealdialysesystem umfasst dabei einen Behälter 10 mit frischem Dialysat und einen Abfluss 20 für gebrauchtes Dialysat. Weiterhin ist ein Konnektor 30 vorgesehen, welcher an einen Katheter des Patienten angeschlossen werden kann, um entweder frisches Dialysat in die Bauchhöhle des Patienten einzuführen oder gebrauchtes Dialysat aus der Bauchhöhle abzuführen. Der Behälter 10 mit frischem Dialysat, der Abfluss 20 für gebrauchtes Dialysat und der Konnektor 30 zum Patienten sind dabei über Fluidwege 100 miteinander verbunden und bilden zusammen mit diesen das Fluidsystem des Peritonealdialysesystems.

Zur Durchführung der Peritonealdialysebehandlung ist eine Dialysemaschine 40, auch Cycler genannt, vorgesehen. Die Dialysemaschine 40 umfasst dabei folgende Hauptkomponenten:
- Eine Pumpe 50, welche zum Transport der Flüssigkeiten eingesetzt wird. Die Pumpe 50 befördert dabei das frische Dialysat vom Behälter 10 zum Konnektor 30. Weiterhin kann die Pumpe 50 das verbrauchte Dialysat vom Konnektor 30 zum Abfluss 20 transportieren.
- Ventile 70, welche zur Steuerung der Flüssigkeitsströme eingesetzt werden. Die Ventile 70 öffnen und schließen die Fluidwege 100, um so die entsprechenden Fluidverbindungen zwischen dem Behälter 10, dem Konnektor 30 und dem Abfluss 20 herzustellen.
- Eine Heizung 60, welche das frische Dialysat auf eine Temperatur von ca. 37°C bringt, bevor dieses dem Patienten zugeführt wird. Da bei der Peritonealdialyse relativ große Mengen an Dialysat direkt in die Bauchhöhle des Patienten eingeführt werden, ist die Heizung 60 nötig, um den Patienten nicht zu unterkühlen und um ein unangenehmes Gefühl durch zu kaltes Dialysat zu vermeiden.
- Sensoren 80, über welche der ordnungsgemäße Ablauf der Behandlung überwacht und/oder gesteuert werden kann. Insbesondere können dabei Temperatursensoren zum Einsatz kommen. Weiterhin können gegebenenfalls Drucksensoren zum Einsatz kommen.

Alle Komponenten der Dialysemaschine 40 werden dabei über eine Steuerung 90 angesteuert. Die Steuerung 90 steuert dabei insbesondere die Pumpe 50, die Heizung 60 und die Ventile 70 auf Grundlage der Daten der Sensoren 80 an. Die Steuerung 90 sorgt dabei für den automatischen Ablauf der Peritonealdialyse. Als wichtige Komponente umfasst die Steuerung 90 dabei eine Bilanzierung 95, welche die dem Patienten zugegebenen und entnommenen Flüssigkeitsmengen bilanziert. Die Bilanzierung verhindert dabei, dass dem Patienten zuviel Flüssigkeit zugegeben oder zuviel Flüssigkeit entnommen wird.

Die Bilanzierung 95 kann dabei allein auf Grundlage der Ansteuerdaten und/oder der Sensordaten für die Pumpe 50 erfolgen. Alternativ kann die Bilanzierung auch über separat vorgesehene Bilanzierkammern erfolgen. Ebenso ist es möglich, zur Bilanzierung eine Waage einzusetzen. Eine solche Waage wiegt beispielsweise das Gewicht des Behälters 10 mit frischem Dialysat und/oder eines Behälters 20 mit gebrauchtem Dialysat.

Da bei der Peritonealdialyse das Dialysat dem Patienten direkt in die Bauchhöhle verabreicht wird, ist auf äußerste Sterilität zu achten. Daher werden die Fluidwege bzw. das Fluidsystem, welches mit dem frischen und/oder dem gebrauchten Dialysat in Kontakt kommt, üblicherweise als Einwegteil ausgeführt. Insbesondere werden die Fluidwege bzw. das Fluidsystem dabei als Kunststoffteile ausgeführt. Diese können so in einer sterilen Umverpackung angeliefert und erst kurz vor der Behandlung ausgepackt werden.

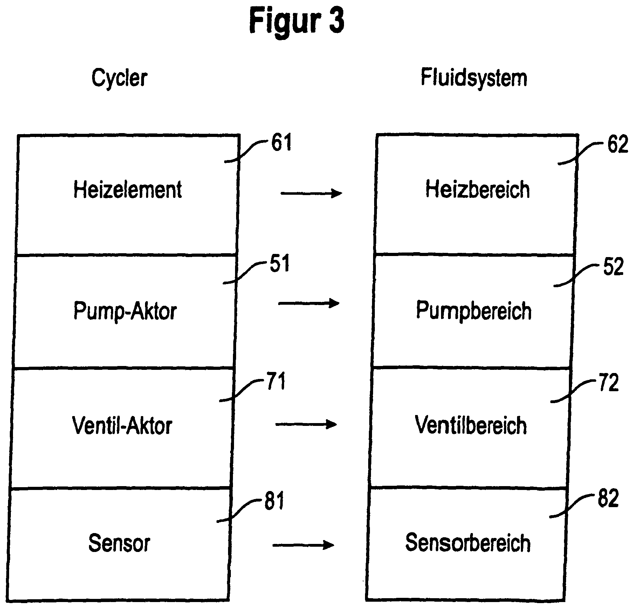

Um dennoch eine Steuerung der Peritonealdialyse durch die Dialysemaschine 40 zu ermöglichen, muss das Fluidsystem an die Dialysemaschine 40 angekoppelt werden. In Figur 3 ist dabei schematisch dargestellt, wie einzelne Elemente der Dialysemaschine 40 an entsprechende Bereiche des Fluidsystems angekoppelt werden.

Die Dialysemaschine 40 weist dabei ein Heizelement 61 auf. Dieses muss an einen entsprechenden Heizbereich 62 des Fluidsytems angekoppelt werden. Die Ankopplung ermöglicht dabei die Übertragung von Wärmeenergie von dem Heizelement 61 an das im Heizbereich 62 befindliche Dialysat.

Die Dialysemaschine 40 weist weiterhin einen oder mehrere Pumpaktoren 51 auf, welche mit einem Pumpbereich 52 des Fluidsystems gekoppelt werden. Die Pumpaktoren 51 erzeugen dabei eine Pumpkraft, welche auf den Pumpbereich 52 übertragen wird. Hierdurch kann die im Pumpbereich 52 befindliche Flüssigkeit entlang der Fluidwege bewegt werden.

Weiterhin weist die Dialysemaschine einen oder mehrere Ventilaktoren 71 auf. Diese erzeugen eine Schließbewegung, welche auf entsprechende Ventilbereiche 72 der Fluidwege übertragen wird. Hierdurch können die Ventilbereiche 72 der Fluidwege entsprechend geschlossen bzw. geöffnet werden.

Weiterhin weist die Dialysemaschine einen oder mehrere Sensoren 81 auf. Diese werden an einen entsprechenden Sensorbereich 82 des Fluidsystems angekoppelt. Hierdurch können die Sensoren 81 gewisse Eigenschaften des Dialysats messen. Insbesondere kann hierdurch die Temperatur des Dialysats gemessen werden. Weiterhin kann vorgesehen sein, dass der Druck in dem Fluidsystem bestimmt wird.

Selbstverständlich weist die Dialysemaschine gegebenenfalls noch weitere Aktoren und/oder Sensoren auf, welche nicht mit den Fluidwegen gekoppelt werden müssen.

Die einzelnen Komponenten eines Peritonealdialysesystems sollen nun im folgenden näher anhand von Ausführungsbeispielen dargestellt werden.

### 1. Fluidsystem

### 1.1 Dialysatbehälter

Frisches Dialysat wird üblicherweise in Kunststoffbeuteln zur Verfügung gestellt. Solche Kunststoffbeutel weisen üblicherweise zwei Lagen von Kunststofffolie auf, welche in einem Randbereich miteinander verschweißt sind und so einen Behälter bilden, welcher mit frischem Dialysat befüllt ist. An diesen Behälter ist üblicherweise ein Schlauchelement angeschweißt, durch welches das Dialysat aus dem Beutel entnommen werden kann. An dem Schlauchelement ist üblicherweise ein Konnektor angeordnet, über welchen der Dialysatbehälter mit den übrigen Fluidwegen verbunden werden kann. Weiterhin weist der Beutel üblicherweise an der dem Schlauch gegenüberliegenden Seite eine Aussparung oder Öse auf, über welche der Beutel an einen Haken aufgehängt werden kann. Hierdurch kann sichergestellt werden, dass das Dialysat problemlos aus dem Beutel abfließt.

Das Dialysat besteht üblicherweise aus einem Puffer, einem Osmotikum und Elektrolyten. Als Puffer kann dabei z. B. Bicarbonat eingesetzt werden. Als Osmotikum wird üblicherweise Glucose eingesetzt. Alternativ können auch Glucosepolymere oder Glucosepolymerderivate eingesetzt werden. Die Elektrolyte umfassen üblicherweise Kalzium und Natrium.

Das Dialysat kann dabei hitzesterilisiert werden. Dies erfolgt vorteilhafterweise, nachdem das Dialysat in den Beutel gefüllt wurde. Hierdurch werden sowohl das Dialysat als auch der Beutel hitzesterilisiert. Dabei wird der gefüllte Beutel üblicherweise zunächst in eine Umverpackung verpackt, woraufhin das gesamte System sterilisiert wird.

Da die fertige Dialysatlösung je nach Inhaltsstoffen oft nicht hitzesterilisiert werden kann oder nicht lange gelagert werden kann, kann vorgesehen sein, einzelne Komponenten des Dialysats getrennt zu lagern und erst kurz vor der Behandlung zusammenzuführen. Eine erste Einzellösung umfasst dabei üblicherweise den Puffer, während eine zweite Einzellösung Glucose und Elektrolyte umfasst. Gegebenenfalls können auch mehr als zwei Einzellösungen und damit mehr als zwei Bereiche in einem Beutel vorgesehen sein. Dabei kann ein Mehrfachkammerbeutel, insbesondere ein Doppelkammerbeutel vorgesehen sein, welcher mehrere getrennte Bereiche zur Lagerung der Einzellösungen aufweist. Diese Bereiche sind durch ein Verbindungselement getrennt, welches mechanisch geöffnet werden kann, um die Einzelflüssigkeiten miteinander zu vermischen. Insbesondere kann dabei eine sogenannte Peel-Naht zwischen den beiden Bereichen des Beutels vorgesehen sein, welche sich bei Anwendung eines bestimmten Druckes auf mindestens einen der Bereiche des Beutels öffnet.

Da während einer nächtlichen Peritonealdialysebehandlung relativ große Mengen an Dialysat verbraucht werden, werden üblicherweise mehrere Dialysatbehälter parallel eingesetzt. Diese werden über entsprechende Konnektoren mit den Fluidwegen verbunden und können durch eine entsprechende Schaltung der Ventile zum Befüllen des Patienten herangezogen werden.

### 1.2 Abfluss

Zum Entsorgen der verbrauchten Dialysierflüssigkeit kann diese entweder sofort in die Kanalisation abgeführt oder zunächst in einem Abflussbehälter gesammelt werden. Als Abflussbehälter wird dabei üblicherweise ebenfalls ein Beutel eingesetzt. Dieser ist vor Beginn der Behandlung leer und kann so das verbrauchte Dialysat aufnehmen. Der Beutel kann dann nach Beendigung der Behandlung entsprechend entsorgt werden.

### 1.3 Kassette

Wie bereits eingangs beschrieben, weist das Fluidsystem eine Mehrzahl von Bereichen auf, in welchen die Dialysemaschine auf das Fluidsystem einwirken muss. Hierfür muss das Fluidsystem an die Dialysemaschine angekoppelt werden.

Um das Ankoppeln der Fluidwege an die Dialysemaschine und die Einwirkung der entsprechenden Elemente der Dialysemaschine auf die Fluidwege zu vereinfachen, werden Kassetten eingesetzt. In einer solchen Kassette sind mehrere Bereiche, in welchen die Dialysemaschine auf die Fluidwege einwirkt, gemeinsam angeordnet. Hierfür weist eine Kassette üblicherweise ein Hartteil aus Kunststoff auf, in welches zu einer Seite hin offene Kammern als Fluidwege eingebracht sind. Diese Kammern werden von einer flexiblen Kunststofffolie bedeckt, welche für die Ankopplung an die Dialysemaschine sorgt. Die flexible Kunststofffolie ist dabei üblicherweise in einem Randbereich mit dem Hartteil verschweißt. Die Kassette wird mit einer Ankoppelfläche der Dialysemaschine verpresst, so dass die Aktoren und/oder Sensoren der Dialysemaschine mit entsprechenden Bereichen der Kassette in Kontakt kommen.

Die Kassette weist weiterhin Anschlüsse zum Anschluss des Dialysatbehälters 10, des Konnektors 30 sowie des Ablaufs 20 auf.

Eine Kassette umfasst dabei üblicherweise zumindest einen Pumpbereich und einen oder mehrere Ventilbereiche. Über die Kassette kann so der Flüssigkeitstransport durch das Fluidsystem gesteuert werden. Weiterhin kann die Kassette Sensorbereiche aufweisen, welche eine einfache Ankopplung von Sensoren der Dialysemaschine an das Fluidsystem ermöglichen. Gegebenenfalls kann die Kassette weiterhin einen oder mehrere Heizbereiche aufweisen, welche an entsprechende Heizelemente der Dialysemaschine ankoppelbar sind.

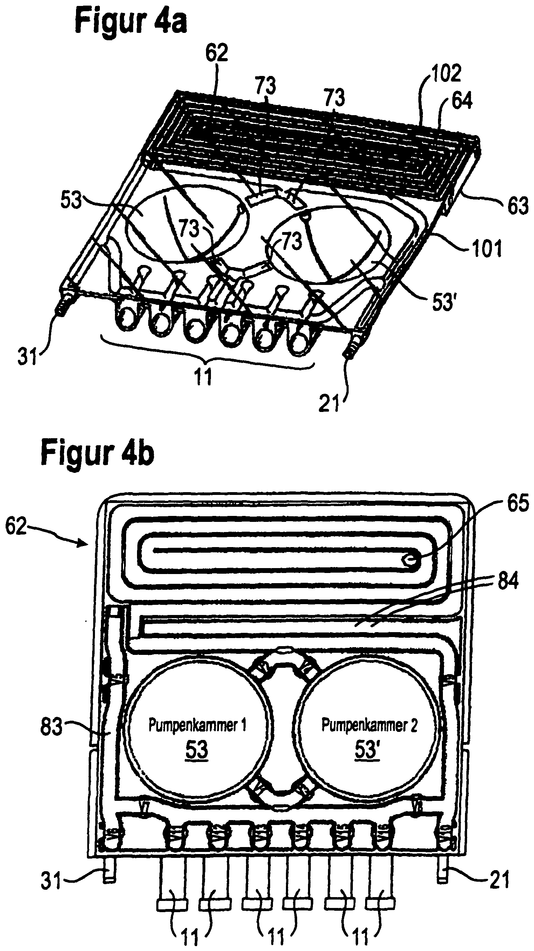

In Figuren 4a und 4b ist ein erstes Ausführungsbeispiel einer Kassette dargestellt. Diese weist ein Hartteil 101 aus Kunststoff auf, in welchem die Fluidwege und Ankopplungsbereiche als entsprechende Aussparungen, Kammern und Kanäle eingebracht sind. Das Hartteil kann dabei z. B. als Spritzgussteil oder als Tiefziehteil gefertigt werden. Die Ankoppelebene des Hartteils 101 wird von einer flexiblen Folie 102 bedeckt, welche in einem Randbereich mit dem Hartteil verschweißt ist. Durch das Verpressen der Kassette mit einer Ankoppelfläche der Dialysemaschine wird die flexible Folie 102 mit dem Hartteil verpresst. Durch das Verpressen der flexiblen Folie mit den Stegbereichen des Hartteils werden die Fluidwege innerhalb der Kassette fluiddicht voneinander getrennt.

Die Kassette weist Anschlüsse zum Anschluss der Kassette an die übrigen Fluidwege auf. Zum Einen ist ein Anschluss 21 zum Anschluss an den Abfluss 20 sowie ein Anschluss 31 zum Anschluss an den Konnektor 30 vorgesehen. An diesen Anschlüssen können entsprechende Schlauchelemente vorgesehen sein, welche in Figur 4a nicht dargestellt sind. Weiterhin weist die Kassette eine Mehrzahl von Anschlüssen 11 zum Anschluss von Dialysatbehältern 10 auf. Die Anschlüsse 11 sind dabei im ersten Ausführungsbeispiel als Konnektoren ausgeführt, an welche entsprechende Konnektorelemente angeschlossen werden können.

Die Anschlüsse stehen jeweils mit Fluidwegen innerhalb der Kassette in Verbindung. In diesen Fluidwegen sind Ventilbereiche vorgesehen. In diesen Ventilbereichen kann die flexible Folie 102 über maschinenseitige Ventilaktoren so in das Hartteil 101 gedrückt werden, dass der entsprechende Fluidweg versperrt ist. Die Kassette weist dabei zunächst einmal für jeden Anschluss ein entsprechendes Ventil auf, über welches dieser Anschluss geöffnet bzw. geschlossen werden kann. Dem Anschluss 21 für den Abfluss 20 ist dabei das Ventil V10 zugeordnet, dem Anschluss 31 für den Patientenkonnektor 30 das Ventil V6. Den Anschlüssen 11 für die Dialysatbehälter 10 sind die Ventile V11 bis V16 zugeordnet.

Weiterhin sind in der Kassette Pumpenkammern 53 und 53' vorgesehen, welche durch entsprechende Pumpaktoren der Dialysemaschine betätigt werden können. Bei den Pumpenkammern 53 und 53' handelt es sich dabei um konkave Aussparungen in dem Hartteil 101, welche von der flexiblen Folie 102 bedeckt werden. Durch Pumpaktoren der Dialysemaschine kann die Folie nun in die Pumpenkammern 53 und 53' hineingedrückt bzw. wieder aus diesen Pumpenkammern herausgezogen werden. Hierdurch kann, im Zusammenspiel mit den Ventilen V1 bis V4, welche die Zugänge und Abläufe der Pumpenkammern 53 und 53' schalten und in Fig. 4a mit dem Bezugszeichen 73 bezeichnet wurden, ein Pumpstrom durch die Kassette erzeugt werden. Die Pumpkammern sind dabei über entsprechende Ventilschaltungen mit allen Anschlüssen der Kassette verbindbar.

Weiterhin ist ein Heizbereich 62 in die Kassette integriert. In diesem Bereich wird die Kassette mit Heizelementen der Dialysemaschine in Kontakt gebracht, welche das durch diesen Bereich der Kassette fließende Dialysat erwärmen. Der Heizbereich 62 weist dabei einen Kanal für das Dialysat auf, welcher sich spiralförmig über den Heizbereich 62 erstreckt. Der Kanal wird dabei durch Stege 64 des Hartteils gebildet, welche von der flexiblen Folie 102 abgedeckt sind.

Der Heizbereich 62 ist dabei auf beiden Seiten der Kassette vorgesehen. Hierfür ist auch auf der Unterseite 63 der Kassette im Heizbereich eine flexible Folie am Hartteil angeordnet. Die flexible Folie ist dabei ebenfalls in einem Randbereich mit dem Hartteil verschweißt. Auf der Unterseite ist ebenfalls ein Kanal angeordnet, durch welchen das Dialysat fließt. Die Kanäle auf der Unterseite und der Oberseite werden dabei durch eine mittlere Platte des Hartteils gebildet, welche die Oberseite von der Unterseite trennt, und auf welcher nach unten und nach oben Stege vorgesehen sind, welche die Kanalwandungen bilden. Dabei fließt das Dialysat zunächst spiralförmig auf der Oberseite bis zum Durchbruch 65 durch die mittlere Platte, von wo aus das Dialysat auf der Unterseite durch den entsprechenden Kanal zurückfließt. Durch den auf der Ober- und der Unterseite vorgesehenen Heizbereich kann die Heizfläche, welche zum Aufheizen der Flüssigkeit zur Verfügung steht, entsprechend vergrößert werden. Selbstverständlich ist aber auch eine Ausführungsform der Kassette möglich, bei welcher nur auf einer Seite der Kassette ein Heizbereich angeordnet ist.

Weiterhin sind Ausführungsformen der Kassette möglich, bei welcher ein Heizelement in die Kassette integriert ist. Insbesondere kann dabei ein elektrisches Heizelement wie z.B. Heizwendel in das Hartteil der Kassette eingegossen sein. Hierdurch kann auf ein maschinenseitiges Heizelement verzichtet werden und die Durchflussheizung in die Kassette integriert werden. Dabei sind an der Kassette elektrische Kontakte zur Konnektierung des elektrischen Heizelements angeordnet.

Die Kassette weist weiterhin Sensorbereiche 83 und 84 auf, durch welche Temperatursensoren der Dialysemaschine an die Kassette gekoppelt werden können. Die Temperatursensoren liegen dabei auf der flexiblen Folie 102 auf und können so die Temperatur der durch den darunter liegenden Kanal fließenden Flüssigkeit messen. Dabei sind am Eingang des Heizbereiches zwei Temperatursensoren 84 vorgesehen. Am patientenseitigen Ausgang ist ein Temperatursensor 83 vorgesehen, über welchen die Temperatur des zum Patienten gepumpten Dialysats gemessen werden kann.

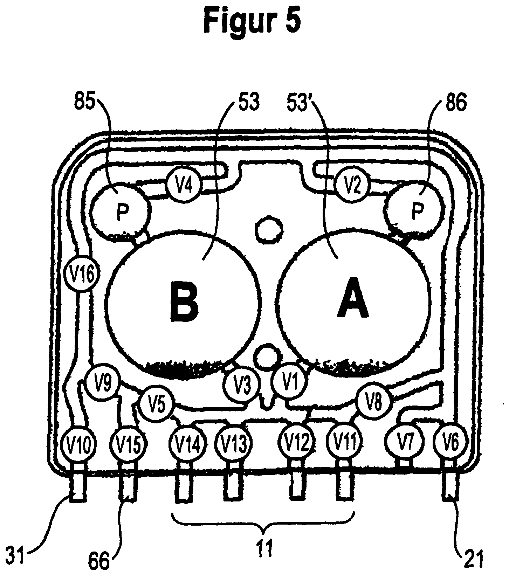

In Figur 5 ist ein zweites Ausführungsbeispiel für eine Kassette gezeigt. Die Kassette entspricht dabei in ihrer Ausführung im Wesentlichen dem ersten Ausführungsbeispiel, umfasst jedoch keinen Heizbereich. Bei Einsatz dieser Kassette erfolgt die Heizung daher nicht wie beim ersten Ausführungsbeispiel gezeigt über einen in die Kassette integrierten Heizbereich, sondern z.B. über einen Heizbeutel, welcher auf eine Heizplatte der Dialysemaschine gelegt wird.

Das in Figur 5 gezeigte zweite Ausführungsbeispiel einer Kassette weist wiederum Fluidwege auf, welche über Ventilbereiche, die hier ebenfalls von V1 bis V16 durchnumeriert sind, geöffnet und geschlossen werden können. Weiterhin weist die Kassette Anschlüsse zum Anschluss an weitere Komponenten des Fluidsystems auf. Dabei ist wiederum der Anschluss 21 zum Anschluss an den Abfluss 20, sowie der Anschluss 31 zum Anschluss an den Konnektor 30 zum Patienten vorgesehen. Weiterhin sind Anschlüsse 11 zum Anschluss von Dialysatbehältern 10 vorgesehen.

Im Unterschied zum ersten Ausführungsbeispiel weist die im zweiten Ausführungsbeispiel gezeigte Kassette einen weiteren Anschluss 66 zum Anschluss eines Heizbeutels auf. Zum Erwärmen der Flüssigkeit aus den Dialysatbehältern 10 kann die Flüssigkeit dabei über den Anschluss 66 in einen Heizbeutel gepumpt werden. Dieser Heizbeutel liegt auf einem Heizelement auf, so dass die im Heizbeutel befindliche Flüssigkeit erwärmt werden kann. Daraufhin wird die Flüssigkeit aus dem Heizbeutel zum Patienten gepumpt.

Die Pumpkammer 53 und 53' und die Ventile V1 bis V4 entsprechen in Aufbau und Funktion den entsprechenden Komponenten im ersten Ausführungsbeispiel.

Im Unterschied zum ersten Ausführungsbeispiel weist die Kassette im zweiten Ausführungsbeispiel keinen Sensorbereich zum Anschluss eines Temperatursensors auf. Dieser ist vielmehr im Bereich der Heizelemente angeordnet. Die Kassette weist jedoch Messbereiche 85 und 86 zum Messen des Drucks in den Pumpekammern 53 und 53' auf. Die Messbereiche 85 und 86 sind dabei Kammern, welche mit dem Pumpkammern fluidisch in Verbindung stehen und ebenfalls von der flexiblen Folie abgedeckt werden. An die Messbereiche können geräteseitige Drucksensoren angekoppelt werden, welche den Druck in den Messkammern 85 und 86 und damit in den Pumpkammern 53 und 53' messen.

Die Verbindung der Anschlüsse 11, 21, 31 und 66 der Kassette mit den weiteren Komponenten des Fluidsystems erfolgt im zweiten Ausführungsbeispiel über Schlauchverbindungen. An diesen Schlauchverbindungen sind gegebenenfalls Konnektoren angeordnet.

### 1.3 Schläuche

Die Verbindung zwischen den einzelnen Behältern des Systems, der Kassette und dem Patientenkonnektor erfolgt üblicherweise über Schlauchverbindungen. Da es sich jeweils um Einwegartikel handelt, sind die Schläuche dabei üblicherweise zumindest auf einer Seite bereits mit einem weiteren Element fest verbunden. Z. B. können Schläuche bereits an einem oder mehreren der Anschlüsse der Kassette vorgesehen sein. Ebenfalls können Schläuche bereits fest mit Beuteln in Verbindung stehen.

### 1.4 Verbindungen

Das Fluidsystem ist üblicherweise in mehrere Teile aufgeteilt und jeweils steril verpackt. Diese Teile müssen für die Behandlung zunächst miteinander verbunden werden. Insbesondere sind dabei üblicherweise die Kassette sowie der oder die Dialysatbeutel getrennt voneinander verpackt.

Die Verbindungen zwischen den einzelnen Elementen des Fluidsystems erfolgt üblicherweise über Konnektoren. Die Konnektoren sind dabei so gestaltet, dass sie eine sterile Verbindung zwischen den einzelnen Komponenten ermöglichen. Dies erfolgt z. B. über entsprechende Schutzfolien, welche beim Schließen des Konnektors automatisch geöffnet werden.

Die Verbindung der einzelnen Komponenten kann dabei manuell durch eine Bedienperson bzw. den Patienten selbst erfolgen. Alternativ kann vorgesehen sein, dass die Verbindung der einzelnen Komponenten durch die Dialysemaschine erfolgt.

Hierzu können z. B. die entsprechenden Konnektoren in eine Konnektoraufnahme der Dialysemaschine eingelegt und automatisch durch die Dialysemaschine zusammengeführt werden.

Weiterhin kann eine elektronische Steuerung vorgesehen sein, welche überwacht, dass die richtigen Komponenten des Systems miteinander verbunden werden. Hierfür können an den Konnektoren Identifikationsmittel wie z. B. Barcodes oder RFIDs vorgesehen sein, welche die Komponenten identifizieren. Die Dialysemaschine umfasst dabei eine Identifikationsmittel-Erfassungseinheit wie z. B. einen Barcodeleser oder eine RFID-Erfassungseinheit, welcher die Identifikationsmittel auf den Konnektoren erfasst. Hierdurch kann die Steuerung der Peritonealdialyse erkennen, ob die korrekten Konnektoren eingelegt wurden.

Eine solche Überprüfung der korrekten Zusammenstellung des Fluidsystems kann dabei insbesondere mit einer automatischen Konnektion der Konnektoren kombiniert sein. Das System überprüft so zunächst, ob die richtigen Konnektoren in die Konnektorenaufnahmen eingelegt wurden. Die Verbindung zwischen den Konnektoren wird durch die Dialysemaschine nur hergestellt, wenn die richtigen Konnektoren eingelegt wurden. Andernfalls macht die Dialysemaschine den Benutzer darauf aufmerksam, dass die falschen Konnektoren eingelegt wurden.

### 2. Die Dialysemaschine

Die einzelnen Komponenten einer Dialysemaschine sollen nun im folgenden anhand von zwei Ausführungsbeispielen näher beschrieben werden.

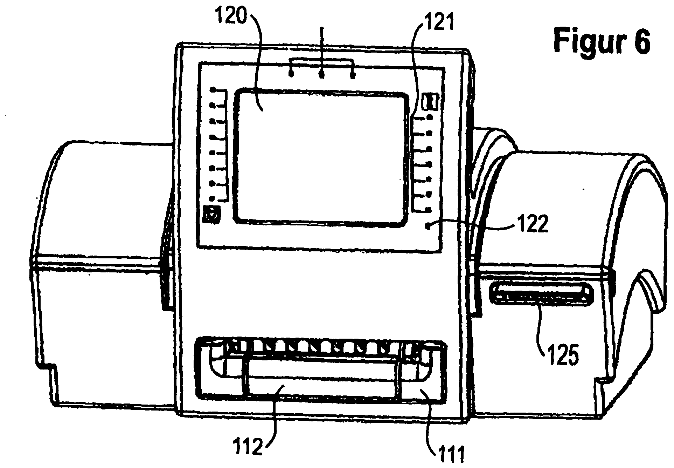

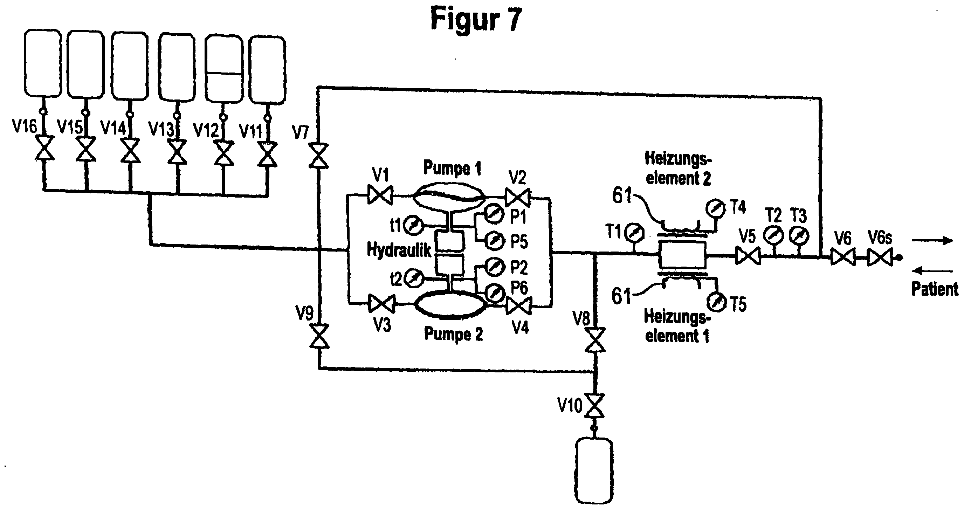

In Figur 6 ist dabei ein erstes Ausführungsbeispiel einer Dialysemaschine gezeigt, bei welchem das erste Ausführungsbeispiel einer Kassette eingesetzt wird. Das sich aus dem ersten Ausführungsbeispiel einer Dialysemaschine und dem ersten Ausführungsbeispiel einer Kassette ergebende Peritonealdialysesystem ist dabei in Figur 7 gezeigt.

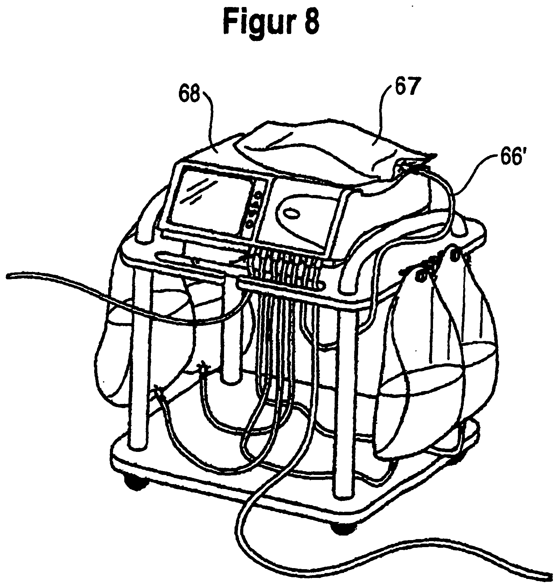

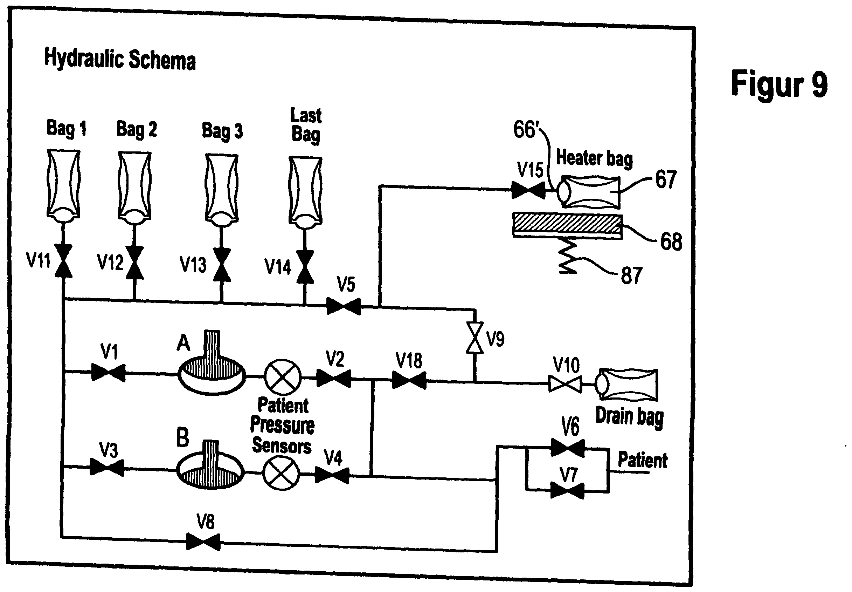

In Figur 8 ist ein zweites Ausführungsbeispiel einer Dialysemaschine gezeigt, bei welcher das zweite Ausführungsbeispiel einer Kassette zum Einsatz kommt. Das sich aus der Kombination des zweiten Ausführungsbeispiels einer Dialysemaschine und des zweiten Ausführungsbeispiels einer Kassette ergebende Dialysesystem ist dann in Figur 9 gezeigt.

Die beiden Ausführungsbeispiele unterscheiden sich dabei zum einen in der Ausgestaltung der Heizung, in der Kopplung zwischen der Dialysemaschine und der Kassette sowie in der Ausgestaltung der Aktoren und Sensoren.

### 2.1 Heizung

Das frische Dialysat muss auf Körpertemperatur gebracht werden, bevor es in den Bauchraum des Patienten befördert wird. Hierfür weist die Dialysemaschine eine entsprechende Heizung auf.

Die Heizung erfolgt dabei üblicherweise elektrisch über ein oder mehrere Heizelemente. Bei den Heizelementen kann es sich dabei z. B. um keramische Heizelemente handeln. Bei solchen keramischen Heizelementen ist eine Widerstandsbahn auf einem Keramikträger aufgebracht. Durch Anlegen einer Spannung an die Widerstandsbahn wird diese erhitzt, wodurch sich auch das keramische Trägermaterial erhitzt. Das keramische Heizelement ist dabei üblicherweise auf einer Heizplatte angeordnet. Diese kann beispielsweise aus Aluminium gefertigt sein. An die Heizplatte werden wiederum die Fluidwege angekoppelt, so dass das in den Fluidwegen befindliche Dialysat erhitzt werden kann.

Zum Erwärmen der Flüssigkeit stehen zwei unterschiedliche Ausgestaltungen zur Verfügung. Zum einen kann zunächst eine größere Menge an Dialysat erwärmt werden, welche erst nach der Aufwärmphase zum Patienten gepumpt wird. Dies erfolgt üblicherweise über einen Heizbeutel, welcher auf einer Heizplatte des Dialysegerätes aufliegt.

Bei dem Heizbeutel kann es sich dabei um den Dialysatbeutel handeln, in welchem das Dialysat zur Verfügung gestellt wird. Üblicherweise wird jedoch ein separater Heizbeutel eingesetzt, in welchem das Dialysat zum Aufheizen hineingepumpt wird. Ist das Dialysat im Heizbeutel erwärmt, wird es von dort aus zum Patienten gepumpt.

Ein solches Konzept ist bei den in Figuren 8 und 9 gezeigten zweiten Ausführungsbeispiel einer Dialysemaschine verwirklicht. Dabei ist ein Heizbeutel 67 vorgesehen, welcher auf einer Heizplatte 68 aufliegt. Die Heizplatte 68 ist dabei auf der Oberseite des Peritonealdialysegerätes angeordnet, so dass sie leicht zugänglich ist. Der Heizbeutel 67 ist dabei über eine Leitung 66' mit der Kassette verbunden. Die Kassette weist dabei die Ventile V5, V9 und V15 auf, über welche der Heizbeutel 67 mit den übrigen Komponenten des Fluidsystems verbunden werden kann. So kann frisches Dialysat von den Dialysatbehältern 10 über die Pumpkammern zum Heizbeutel 67 gepumpt werden. Zu Beginn einer Behandlung wird also zunächst der Heizbeutel 67 mit kaltem Dialysat gefüllt. Das Dialysat im Heizbeutel 67 wird dann über die Heizplatte 68 auf Körpertemperatur erwärmt. Daraufhin wird das Dialysat über die Pumpkammern zum Patienten gepumpt. Daraufhin kann der Heizbeutel 67 wieder befüllt werden, so dass die für den nächsten Behandlungszyklus benötigte Dialysatmenge erhitzt werden kann.

Vorteilhafterweise ist dabei im Bereich der Heizplatte 68 ein Temperatursensor 88 vorgesehen, welcher mit dem Heizbeutel 67 in Kontakt steht und so die Temperatur des Dialysats im Heizbeutel 67 messen kann. Weiterhin kann ein Temperatursensor an der Heizplatte oder am Heizelement vorgesehen sein, welcher die Temperatur des Heizelements oder der Heizplatte misst. Eine entsprechende Steuerung sorgt nun dafür, dass die Heizplatte nicht zu heiß für das Material des Beutels wird.

Der Heizbeutel 67 kann zudem Funktionen bei der Billanzierung der Flüssigkeitsströme übernehmen. So kann die Heizplatte 68 Teil einer Waage 87 sein, über welche das Gewicht des Heizbeutels 67 bestimmbar ist. Hierdurch kann die Flüssigkeitsmenge, welche nach dem Erwärmen dem Patienten zugeführt wird, bestimmt werden.

Alternativ zu der beim zweiten Ausführungsbeispiel gezeigten Erwärmung des Dialysats über einen Heizbeutel kann das Dialysat auch erwärmt werden, während es zum Patienten gepumpt wird. Die Heizung arbeitet damit in Form eines Durchlauferhitzers, welcher das durch das Fluidsystem bewegte Dialysat erwärmt, während es durch die Fluidwege gepumpt wird.

Bei diesem Konzept ist ein Dialysatkanal vorgesehen, welcher an ein Heizelement der Dialysemaschine gekoppelt wird. Während das Dialysat durch den Dialysatkanal fließt, nimmt es dabei Wärme von dem Heizelement der Dialysemaschine auf.

Ein solches Konzept ist bei dem ersten Ausführungsbeispiel einer Dialysemaschine, welches in Figuren 6 und 7 gezeigt ist, implementiert. Dabei ist der Heizbereich in die Kassette integriert, wie dies bereits oben dargestellt wurde. Beim Ankoppeln der Kassette an die Dialysemaschine kommt dabei der Heizbereich der Kassette mit Heizelementen der Dialysemaschine thermisch in Kontakt.

Die Heizelemente können dabei ebenfalls als keramische Heizelemente ausgeführt sein und mit Heizplatten in Kontakt stehen, welche dann an den Heizbereich der Kassette gekoppelt werden. Wie bereits hinsichtlich der Kassette dargestellt, steht dabei sowohl mit der Oberseite als auch mit der Unterseite des Heizbereichs jeweils eine Heizplatte in Kontakt, welche das durch den Heizbereich fließende Dialysat erwärmt.

Am Zulauf und am Ablauf des Heizbereichs sind jeweils Temperatursensorbereiche in der Kassette vorgesehen, welche durch das Ankoppeln der Kassette mit Temperatursensoren der Peritonealdialyse in Kontakt kommen. Durch die Temperatursensoren T1 bis T3 kann so die Temperatur des in den Heizbereich einfließenden Dialysats sowie die Temperatur des aus dem Heizbereich herausfließenden Dialysats bestimmt werden. Weiterhin sind Temperatursensoren T4 und T5 vorgesehen, welche die Temperatur der Heizelemente und/oder der Heizplatten bestimmen.

Die Verwendung von mindestens zwei Heizelementen ermöglicht es dabei, die Heizelemente jeweils so zusammenzuschalten, dass sie bei einer Versorgungsspannung von 220 V im wesentlichen die gleiche Leistung abgeben wird wie bei einer Versorgungsspannung von 110 V. Hierfür werden die beiden Heizelemente bei 110 V in einer Parallelschaltung betrieben, während sie bei einer Versorgungsspannung von 220 V in einer Seriellschaltung betrieben werden. Eine solche Anpassung der Verschaltung der Heizelemente an die Versorgungsspannung kann dabei unabhängig davon, ob die Heizung gemäß dem ersten oder dem zweiten Ausführungsbeispiel erfolgt, implementiert werden.

### 2.2 Ankopplung der Kassette

Um eine Ankopplung der Aktoren und/oder Sensoren der Dialysemaschine an die entsprechenden Bereiche der Kassette zu ermöglichen, weist die Dialysemaschine eine Kassettenaufnahme mit einer Ankoppelfläche auf, an welcher die Kassette angekoppelt werden kann. An der Ankoppelfläche sind die entsprechenden Aktoren, Sensoren und/oder Heizelemente der Dialysemaschine angeordnet. Die Kassette wird mit dieser Ankoppelfläche so verpresst, dass die entsprechenden Aktoren, Sensoren und/oder Heizelemente mit den entsprechenden Bereichen an der Kassette in Kontakt kommen.

Dabei ist vorteilhafterweise an der Ankoppelfläche der Dialysemaschine eine Matte aus einem flexiblen Material vorgesehen, insbesondere eine Silikonmatte. Diese sorgt dafür, dass die flexible Folie der Kassette mit den Stegbereichen der Kassette verpresst wird und so die Fluidwege innerhalb der Kassette voneinander trennt.

Vorteilhafterweise ist weiterhin ein umlaufender Rand der Ankoppelfläche vorgesehen, welcher mit dem Randbereich der Kassette verpresst wird. Vorteilhafterweise erfolgt die Verpressung dabei luftdicht, so dass zwischen der Ankoppelfläche und der Kassette ein Unterdruck aufgebaut werden kann.

Gegebenenfalls kann auch ein Vakuumsystem vorgesehen sein, welches Luft aus dem Raum zwischen Ankoppelfläche und Kassette abpumpen kann. Hierdurch wird eine besonders gute Ankopplung der Aktoren, Sensoren und/oder Heizelemente des Peritonealdialysegerätes mit den entsprechenden Bereichen der Kassette ermöglicht. Zudem erlaubt das Vakuumsystem eine Dichtigkeitsprüfung der Kassette. Hierfür wird nach dem Ankoppeln ein entsprechendes Vakuum angelegt und überprüft, ob dieses Vakuum aufrechterhalten wird.

Das Anpressen der Kassette erfolgt z. B. pneumatisch. Hierzu ist üblicherweise ein Luftkissen vorgesehen, welches mit Druckluft befüllt wird und so die Kassette an die Ankoppelfläche anpresst.

Die Kassettenaufnahme weist üblicherweise eine der Ankoppelfläche gegenüberliegende Aufnahmefläche auf, in welche das Hartteil der Kassette eingelegt wird. Die Aufnahmefläche weist hierfür vorteilhafterweise entsprechende Vertiefungen auf. Die Aufnahmefläche mit der eingelegten Kassette kann dann über eine pneumatische Anpressvorrichtung an die Ankoppelfläche angepresst werden.

Das Einlegen der Kassette kann dabei in unterschiedlicher Weise geschehen. Im ersten Ausführungsbeispiel einer Dialysemaschine, welches in Figur 6 gezeigt ist, ist hierfür eine Schublade 111 vorgesehen, welche aus der Dialysemaschine ausgefahren werden kann. In diese Schublade wird die Kassette eingelegt. Die Kassette wird dann zusammen mit der Schublade in die Dialysemaschine eingeschoben. Daraufhin erfolgt das Verpressen der Kassette mit der Ankoppelfläche, welche im Inneren des Gerätes angeordnet ist. Dabei werden Kassette und Koppelfläche zunächst mechanisch aneinander gefahren und dann pneumatisch miteinander verpresst.

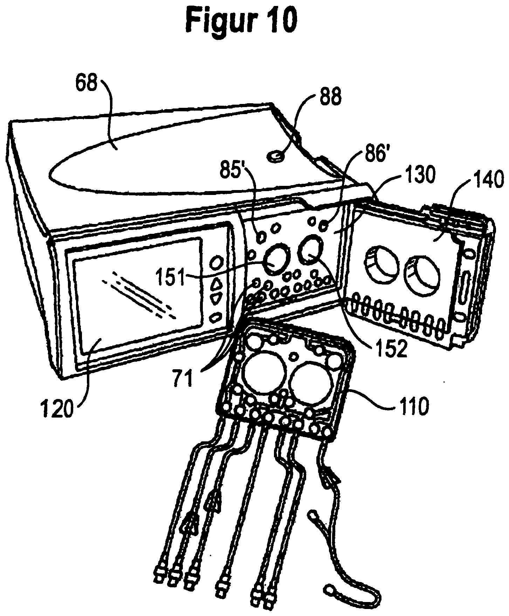

Das Ankoppeln einer Kassette 110 gemäß dem zweiten Ausführungsbeispiel ist in Figur 10 näher dargestellt. Die Ankoppelfläche 130 ist durch Öffnen einer Tür 140 frei zugänglich, so dass die Kassette in der richtigen Position an der Ankoppelfläche 130 angeordnet werden kann. Die Ankoppelfläche 130 ist dabei nach hinten zur Vertikalen geneigt, was ein leichteres Ankoppeln ermöglicht. Nun kann die Tür 140 geschlossen werden, so dass eine Aufnahmefläche an der Tür mit der Rückseite der Kassette in Kontakt kommt. Das Verpressen erfolgt nun durch ein an der Tür angeordnetes Luftkissen. Zudem wird ein Vakuum zwischen die Ankoppelfläche und die Kassette 110 angelegt.

Das erste Ausführungsbeispiel einer Dialysemaschine weist weiterhin eine Vorrichtung zum automatischen Konnektieren auf. Hierfür ist eine Konnektorenaufnahme 112 vorgesehen, in welche die Konnektoren der Dialysatbeutel 10 eingelegt werden. Die Konnektorenaufnahme 112 fährt dann in das Gerät hinein, wo ein Barcodeleser vorgesehen ist, welcher die auf den Konnektoren aufgebrachten Barcodes liest. So kann das Gerät überprüfen, ob die richtigen Beutel eingelegt wurden. Werden die richtigen Beutel erkannt, so fährt die Konnektoraufnahme 112 komplett ein und schließt so die Konnektoren der Beutel an den als Konnektoren ausgeführten Anschlüssen 11 der Kassette an.

Im zweiten Ausführungsbeispiel wurde auf eine solche automatische Konnektierung dagegen verzichtet. Daher sind an den Anschlüssen 11 der Kassette Schlauchabschnitte angeordnet, welche manuell über Konnektoren mit den entsprechenden Beuteln verbunden werden müssen.

### 2.3 Pumpaktoren

Das Pumpen der Flüssigkeit durch das Fluidsystem erfolgt in den Ausführungsbeispielen durch eine Membranpumpe, welche von den Pumpkammern 53 und 53' zusammen mit der flexiblen Folie der Kassette gebildet wird. Wird die flexible Folie dabei durch einen entsprechenden Pumpaktor in die Pumpkammer hineingedrückt, so wird Flüssigkeit aus der Pumpkammer in die geöffneten Bereiche der Fluidwege der Kassette gepumpt. Umgekehrt wird durch Herausziehen der Folie aus der Pumpkammer Fluid aus den Fluidwegen in die Pumpkammer gesaugt.

Der Pumphub erfolgt dabei durch Bewegen eines Pumpaktors in die Pumpkammer. Für den Saughub wird der Pumpaktor wieder von der Pumpkammer wegbewegt. Durch die luftdichte Verpressung von Kassette und Ankoppelfläche entsteht dabei ein Unterdruck, durch welchen die flexible Folie der Kassette dem Pumpaktor folgt und so wieder aus der Pumpkammer herausgezogen wird.

Um eine gute Ankopplung des Pumpaktors an die flexible Folie der Kassette zu ermöglichen, kann zudem ein Vakuumsystem vorgesehen sein. Über das Einstellen eines entsprechenden Vakuums zwischen der Ankoppelfläche und der Kassette kann dabei insbesondere die Kraft eingestellt werden, mit welcher die flexible Folie während eines Saughubs maximal von der Pumpkammer wegbewegt wird.

Hierdurch kann die Saugkraft der Pumpe sehr fein eingestellt werden. Die Pumpkraft wird dagegen durch die Schubkraft des Aktors eingestellt.

Die Bilanzierung der Flüssigkeitsströme kann dabei durch das Zählen der Saug-und Pumphübe erfolgen, da die Membranpumpe eine hohe Genauigkeit der mit jedem Hub gepumpten Flüssigkeitsmenge aufweist.

### 2.3.1. Hydraulischer Antrieb

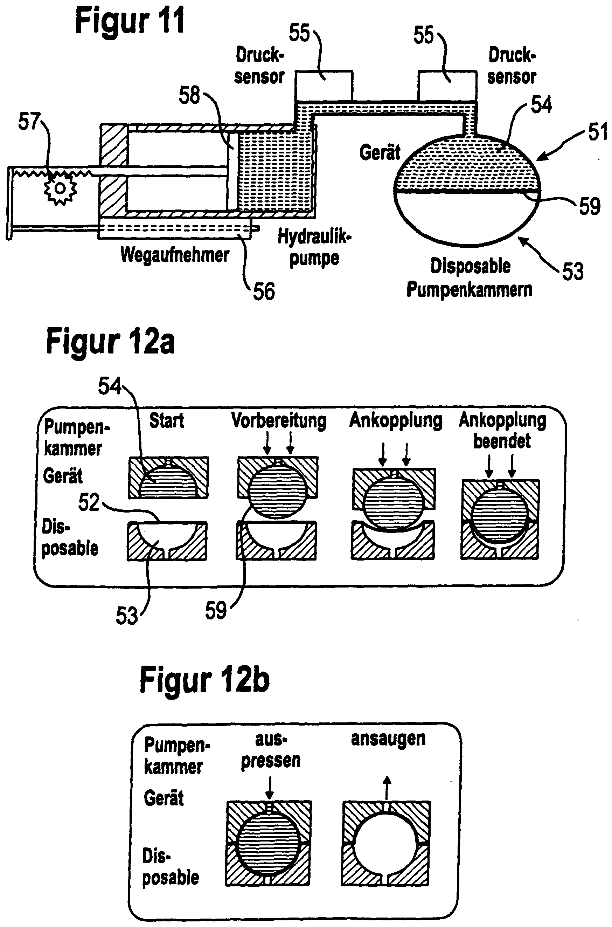

Der Aufbau eines ersten Ausführungsbeispiels eines Pumpaktors ist in Figur 11 gezeigt. Der Pumpaktor wird dabei hydraulisch bewegt. Hierfür ist eine Membran 59 vorgesehen, welche an der flexiblen Folie der Kassette anlegt. Die Membran 59 kann dabei z. B. aus Silikon gefertigt sein. Hinter der Membran 59 ist eine Kammer 54 vorgesehen, welche mit Hydraulikfluid gefüllt werden kann. Durch Anlegen eines Überdrucks in der Kammer 54 wird die Membran 59 und mit dieser die flexible Folie in die Pumpkammer 53 der Kassette hineingedrückt. Durch Anlegen eines Unterdruckes an die Kammer 54 wird die Membran 59 dagegen in die Kammer 54 hineingezogen. Durch den Unterdruck zwischen der flexiblen Folie und der Membran folgt die flexible Folie dieser Bewegung, so dass sich das Volumen der Pumpkammer 53 vergrößert. Der Pumpvorgang mit den Pumphub und dem Ansaughub ist dabei schematisch in Figur 12b dargestellt.

Zum Betrieb der Pumphydraulik ist eine Hydraulikpumpe 58 vorgesehen. Diese weist einen Zylinder auf, in welchem ein Kolben über einen Motor 57 hin und her bewegt werden kann. Hierdurch wird die Hydraulikflüssigkeit über eine entsprechende Verbindungsleitung in die Kammer 54 hineingepresst oder aus dieser wieder herausgesogen. An der Hydraulikpumpe 58 ist dabei ein Wegaufnehmer 56 vorgesehen, über welchen die Bewegung des Kolbens aufgenommen werden kann. Hierdurch kann bestimmt werden, wieviel Hydraulikflüssigkeit in die Kammer 54 hineingepresst bzw. wieviel Hydraulikflüssigkeit aus dieser entnommen wurde. Weiterhin sind Drucksensoren 55 an der Hydraulik vorgesehen, welche den Druck in dem Hydrauliksystem messen. Diese ermöglichen zum einen eine Funktionsüberprüfung der Hydraulik, da die Daten der Drucksensoren mit denen des Wegaufnehmers 56 verglichen werden können und hierdurch die Dichtigkeit des Hydrauliksystems überprüft werden kann.

Zudem ermöglichen die Drucksensoren eine Bestimmung des Drucks in der Pumpekammer 53 der Kassette. Wird die Hydraulikpumpe 58 nicht bewegt, so stellt sich ein Druckgleichgewicht zwischen der Kammer 54 und der Pumpkammer 53 ein. Der Druck der Hydraulikflüssigkeit entspricht damit dem Druck in der Pumpenkammer 53.

In Figur 12a ist nun der Ankoppelvorgang des Pumpenaktors an die Pumpenkammer 53 gezeigt. Zur Vorbereitung des Ankoppelns wird dabei zunächst die Kammer 54 mit Hydraulikfluid beaufschlagt, dass sich die Membran 59 nach außen wölbt. Daraufhin werden Ankoppelfläche und Kassette aufeinander zu bewegt, so dass die Membran 59 die flexible Folie der Kassette in die Pumpkammer 53 hineindrückt. Nach dem Verpressen von Ankoppelfläche und der Kassette ist der Raum zwischen der Membran und der flexiblen Folie nach außen luftdicht abgeschlossen, so dass die flexible Folie der Bewegung der Membran folgt. Dies ist in Figur 12b gezeigt.

Der in Figur 11 gezeigte Pumpaktor ist dabei beim ersten Ausführungsbeispiel einer Dialysemaschine implementiert, wie dies auch aus Figur 7 ersichtlich ist. Dabei ist für jede der beiden Pumpkammern 53 und 53' jeweils ein entsprechender Pumpaktor vorgesehen.

### 2.3.2 Elektromechanischer Antrieb

Alternativ kann der Pumpaktor auch elektromotorisch betrieben werden. Hierfür ist ein entsprechend geformter Stempel vorgesehen, welcher über einen Elektromotor, insbesondere über einen Schrittmotor gegen die flexible Folie gedrückt bzw. von dieser wegbewegt wird und so den Pump- bzw. Saughub erzeugt. Solche Pumpaktoren 151 und 152 sind bei dem Ausführungsbeispiel in Fig. 10 gezeigt. Vorteilhafterweise ist dabei ein Vakuumsystem vorgesehen, welches dafür sorgt, dass die flexible Folie dem Stempel auch bei der Saugbewegung folgt.

### 2.4 Ventilaktoren

Als Ventilaktor kann ein Ventilstößel vorgesehen sein, welcher die flexible Folie der Kassette in eine entsprechende Kammer des Hartteils hineindrückt und so den Fluidkanal in diesem Bereich schließt. Der Ventilaktor kann dabei Z.B. pneumatisch betätigt werden. Der Stößel kann dabei über eine Feder vorgespannt sein, so dass er entweder drucklos öffnet oder drucklos schließt.

Alternativ kann der Ventilaktor über eine flexible Membran implementiert werden, welche hydraulisch oder pneumatisch bewegt wird. Die flexible Membran wird dabei durch Anlegen von Druck gegen die Kassette bewegt und drückt so einen entsprechenden Ventilbereich der flexiblen Folie in einen Fluidkanal, um diesen zu verschließen.

Ventilaktoren 71, welche an die Ventilbereiche V1 bis V16 der Kassette angekoppelt werden, sind in Fig. 10 auf der Ankoppelfläche erkennbar.

### 2.5 Sensoren

Die Dialysemaschine weist Sensoren auf, über welche die Maschine angesteuert bzw. deren ordnungsgemäßes Funktionieren überwacht werden kann.

Zum Einen sind dabei ein oder mehrere Temperatursensoren vorgesehen, über welche die Temperatur des Dialysats und/oder der Heizelemente gemessen werden kann. Im ersten Ausführungsbeispiel sind die Temperatursensoren dabei an der Ankoppelfläche zur Kassette angeordnet und können so die Temperatur des durch die Kassette fließenden Dialysats messen. Im zweiten Ausführungsbeispiel ist dagegen ein Temperatursensor 88 auf der Heizplatte 68 vorgesehen, welcher die Temperatur des im Beutel 67 befindlichen Dialysats misst. Weiterhin können an dem oder den Heizelementen Temperatursensoren vorgesehen sein.

Weiterhin können ein oder mehrere Drucksensoren vorgesehen sein, um den Druck in den Pumpkammern zu bestimmen. Hierdurch kann verhindert werden, das Dialysat mit zu hohem Druck zum Patienten gepumpt wird oder der Saugdruck beim Absaugen von Dialysat vom Patienten zu hoch wird.

Im ersten Ausführungsbeispiel erfolgt die Druckmessung dabei über Drucksensoren in der Hydraulik der Pumpaktoren, wie dies oben dargestellt wurde. Im zweiten Ausführungsbeispiel sind dagegen Drucksensoren 85' und 86' in der Ankoppelfläche vorgesehen, welche den Druck in entsprechenden Druckmessbereichen der Kassette direkt messen. Die Ankopplung dieser Drucksensoren an die Kassette wird dabei vorteilhafterweise durch ein Vakuumsystem sichergestellt.

### 2.6 Ein-/Ausgabeeinheit

Die Dialysemaschine umfasst weiterhin eine Ein-/Ausgabeeinheit zur Kommunikation mit einem Bediener. Zur Ausgabe von Informationen ist dabei eine entsprechende Anzeige vorgesehen, welche z. B. durch Leuchtdioden, LCD-Anzeigen oder einen Bildschirm implementiert sein kann. Zur Eingabe von Befehlen sind entsprechende Eingabeelemente vorgesehen. Hierbei können z. B. Drucktasten und Schalter vorgesehen sein.

In beiden Ausführungsbeispielen ist dabei ein Touchscreen 120 vorgesehen, welcher eine interaktive Menüführung ermöglicht. Weiterhin sind Anzeigeelemente 121 und 122 vorgesehen, welche Zustände der Dialysemaschine kompakt darstellen.

Das erste Ausführungsbeispiel weist weiterhin einen Kartenleser 125 auf, über welchen eine Patientenkarte eingelesen werden kann. Auf der Patientenkarte können Daten zur Behandlung des jeweiligen Patienten abgespeichert sein. Hierdurch kann der Behandlungsablauf für den jeweiligen Patienten individuell festgelegt werden.

Die Peritonealdialysemaschine weist weiterhin eine akustische Signaleinheit auf, über welche akustische Signale abgegeben werden können. Insbesondere kann dabei ein akustisches Warnsignal ausgegeben werden, wenn ein Fehlzustand registriert wird. Dabei ist vorteilhafterweise ein Lautsprecher vorgesehen, über welchen die akustischen Signale erzeugt werden können.

### 2.7 Steuerung

Die Peritonealdialysemaschine weist weiterhin eine Steuerung auf, durch welche sämtliche Komponenten angesteuert und überwacht werden. Die Steuerung sorgt dabei für den automatischen Ablauf der Behandlung.

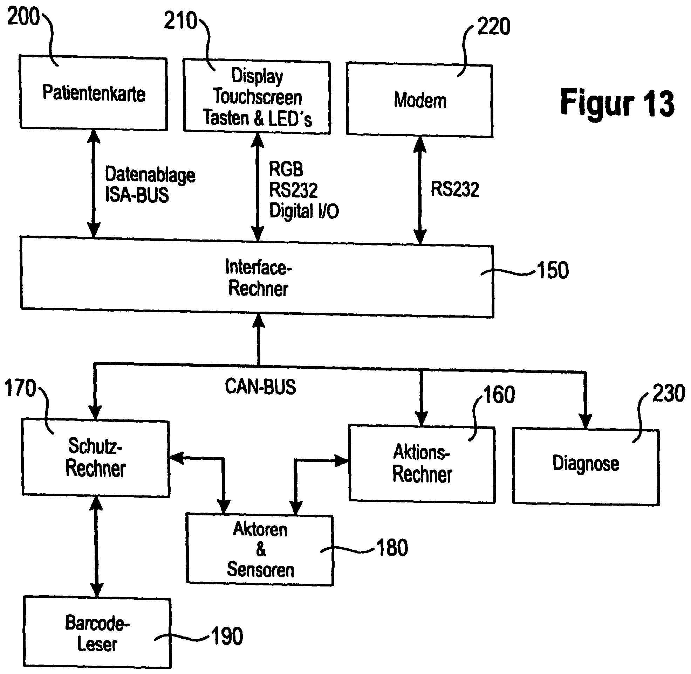

In Figur 13 ist nun der prinzipielle Aufbau eines Ausführungsbeispiels einer solchen Steuerung dargestellt.

Die Kommunikation mit dem Bediener sowie mit externen Informationsquellen erfolgt dabei über einen Interface-Rechner 150. Dieser kommuniziert mit einem Patientenkartenleser 200, einer Ein- und Ausgabeeinheit 210, welche der Kommunikation mit dem Patienten dient, sowie mit einem Modem 220. Über das Modem 220 kann z. B. eine aktualisierte Software aufgespielt werden.

Der Interface-Rechner 150 steht über einen internen Bus mit einem Aktionsrechner 160 und einem Schutzrechner 170 in Verbindung. Der Aktionsrechner 160 und der Schutzrechner 170 erzeugen eine Redundanz des Systems. Der Aktionsrechner 160 erhält dabei Signale von den Sensoren des Systems und berechnet die Steuersignale für die Aktoren 180. Der Schutzrechner 170 erhält ebenfalls Signale von den Sensoren 180 und überprüft, ob die vom Aktionsrechner 160 ausgegebenen Befehle korrekt sind. Stellt der Schutzrechner 170 einen Fehler fest, so leitet er eine entsprechende Notfallprozedur ein. Insbesondere kann der Schutzrechner 170 dabei ein Alarmsignal auslösen. Weiterhin kann der Schutzrechner 170 den Zugang zum Patienten schließen. Hierfür ist ein spezielles Ventil am patientenseitigen Ausgang der Kassette angeordnet, auf welches nur der Schutzrechner 170 Zugriff hat. Dieses Sicherheitsventil ist dabei im drucklosen Zustand geschlossen, so dass es bei einem Ausfall der Pneumatik automatisch schließt.

Der Schutzrechner 170 steht weiterhin mit dem Barcodeleser 190 in Verbindung, und überprüft so den Anschluss der korrekten Dialysatbeutel.

Weiterhin ist ein Diagnosesystem 230 vorgesehen, über welches Fehler des Systems ermittelt und behoben werden können.

### 3. Implementierung der Erfindung

Ein Ausführungsbeispiel der vorliegenden Erfindung, welches bei einem oben dargestellten Dialysesystem bzw. bei einer der oben dargestellten Dialysemaschinen zum Einsatz kommt, wird nun im Folgenden dargestellt. Dabei kann das Ausführungsbeispiel der vorliegenden Erfindung mit einzelnen oder mehreren Komponenten, wie sie oben beschrieben wurden, kombiniert werden.

Die Dialysemaschine weist ein Einstellmittel auf, mittels dessen automatisch wenigstens ein Behandlungsparameter dynamisch und während des Behandlungsbetriebes der Dialysemaschine einstellbar ist.

Dabei weist das Einstellmittel sowohl ein Zeitmanagerelement, auch Zeitmanager genannt, und als auch ein Volumenmanagerelement, auch Volumenmanager genannt, auf. Das Einstellmittel ist vorteilhafterweise Bestandteil der vorstehend beschriebenen Dialysemaschine.

Der Zeitmanager beurteilt vor jeder Verweilphase, ob die Behandlungsdauer um einen festgelegten Faktor (z. B. 10 %) gekürzt werden muss. Da über die durchgeführten Behandlungsphasen Buch geführt wird bzw. dies maschinenseitig protokolliert wird, kann vor jeder Verweilphase beurteilt werden, ob der momentane Zeitplan der Behandlung eingehalten wird.

Falls maschinenseitig eine Verzögerung erkannt wird, wird zunächst die Zeitdifferenz von geplanter zu tatsächlicher Behandlungsdauer ermittelt. Diese Zeitdifferenz (Behandlungsverzögerung) wird von der verschriebenen Verweilphasendauer abgezogen, wenn sie eine festgelegte Kürzungszeit nicht überschreitet (z. B. Verzögerung < 10 % der Verweilphasendauer). Eine Überschreitung der maximal erlaubten Zeit zur Kürzung einer Verweilzeit hat zur Folge, dass die Verweilphase um genau diese maximal festgelegte Zeit gekürzt wird.

Dies lässt sich anhand eines ersten Beispiels erläutern. Demnach soll eine Verweilzeit von 100 Minuten erfolgen. Die momentane Behandlungsverzögerung beträgt in diesem Fall bereits 5 Minuten. Folglich wird die Verweilzeit auf 95 Minuten gekürzt. Dies entspricht einer prozentualen Kürzung von 5 %. Da die prozentuale Kürzung den prozentualen Faktor von 10 % unterschreitet, folgt mittels des Einstellmittels eine Anpassung der Verweilphasendauer auf 95 Minuten.

Zur Erläuterung soll noch ein weiteres Beispiel gegeben werden. Demnach soll eine Verweilzeit von 100 Minuten erfolgen. Hier beträgt die momentane Behandlungsverzögerung bereits 20 Minuten. Demnach beträgt die Behandlungsverzögerung bezogen auf die Verweilphasendauer bereits 20 %. Da die prozentuale Kürzung den prozentualen Faktor um 10 % überschreiten wird, wird seitens des Einstellmittels mit dem Begrenzungsmittel die Verweilphasendauer auf 90 Minuten (90 % der verschriebenen Verweilphasendauer, wobei die Kürzung 10 % der Verweilphasendauer entspricht) festgelegt.

Der Volumenmanager beurteilt vor jedem Einlauf, ob das Einlaufvolumen um einen festgelegten Faktor (z. B. 10 % gekürzt werden muss). Da über das momentan verfügbare Einlaufvolumen und über die restlichen, noch zu durchlaufenden Behandlungsphasen bzw. Behandlungszyklen permanent seitens der Dialysemaschine Protokoll geführt wird, kann vor jedem Einlauf beurteilt werden, ob eine bestimmte Lösungsart für die Durchführung der nachfolgenden Einlaufphasen in ausreichender Menge bzw. mit ausreichendem Volumen vorhanden ist.

Falls das Einlaufvolumen der Lösung nicht ausreicht, um die Behandlung wie verschrieben durchzuführen, erfolgt vorteilhafterweise eine Kürzung sämtlicher noch ausstehender und geplanter Einlaufvolumina, wobei zunächst das fehlende Volumen ermittelt wird.

Hierzu wird in einem ersten Schritt das fehlende Volumen vom verschriebenen Einlaufvolumen dieser Einlaufphase abgezogen. Unterschreitet das fehlende Einlaufvolumen den festgelegten Mindest-Faktor (z. B. < 10 %), wird dieses Volumen vom Einlauf abgezogen. Eine Überschreitung des prozentualen Faktors hat zur Folge, dass der Einlauf um genau diesen prozentualen Faktor gekürzt wird.

Dies soll anhand eines ersten und zweiten Beispiels näher verdeutlicht werden.

Nach dem ersten Beispiel soll ein Einlauf von 1000 ml erfolgen. Hier werden noch 2000 ml einer Lösung für die Durchführung der weiteren Behandlung benötigt. Es stehen jedoch nur noch 1980 ml zur Verfügung. Das Fehlvolumen beträgt somit 20 ml. Der Einlauf wird nun auf 980 ml (= 1000 ml - 20 ml) gekürzt. Dies entspricht einer prozentualen Kürzung von 2 %. Da die prozentuale Kürzung den prozentualen Faktor (10 %) unterschreitet, wird maschinenseitig veranlasst, dass 980 ml einlaufen.

Nach dem zweiten Beispiel soll ebenfalls ein Einlauf von 1000 ml erfolgen. Auch hier werden noch 2000 ml einer Lösung für die Durchführung der weiteren Behandlung benötigt. Es stehen jedoch nur noch 1800 ml zur Verfügung. Das Fehlvolumen beträgt somit 200 ml. Der Einlauf wird nun auf 800 ml (= 1000 ml - 200 ml) gekürzt. Dies entspricht einer prozentualen Kürzung von 20 %. Da die prozentuale Kürzung den prozentualen Faktor von 10 % überschreitet, wird maschinenseitig veranlasst, dass 900 ml einlaufen, was 90 % vom verschriebenen Einlaufvolumen bzw. einer Kürzung von 10 % entspricht.

### Bezugszeichenliste

Behandlungszyklen 1
Einlaufphase 2
Basiseinlaufphase 2'
Verweilphase 3
Auslaufphase 4
Basisauslaufphase 4'
initialer Auslauf 5
Auslaufphase 5'
letzter Einlauf 6
Einlaufphase 6'
Basiszyklus 7
Tidalzyklen 8
Nacht 9
Behälter 10
Anschlüsse 11 zum Anschluss von Behältern 10
Abfluss 20
Anschluss 21 zum Anschluss an den Abfluss 20
Konnektor 30
Anschluss 31 zum Anschluss an den Konnektor 30
Dialysemaschine 40
Pumpe 50
Pumpaktoren 51
Pumpbereich 52
Pumpenkammern 53 und 53'
(Hydraulik)Kammer 54
Drucksensoren 55
Wegaufnehmer 56
Motor 57
Hydraulikpumpe 58
Membran 59
Heizung 60
Heizelement 61
Heizbereich 62
Unterseite des Heizbereichs 63
Stege 64
Durchbruch 65
Anschluss 66 für einen Heizbeutel 67
Leitung 66'
Heizbeutel 67
Heizplatte 68
Ventile 70
Ventilaktoren 71
Ventilbereiche 72
Ventilbereiche 73 für die Pumpkammern
Ventilbereiche V1 bis V16
Sensoren 80
Sensoren 81
Sensorbereiche 82
Sensorbereiche 83 und 84 der Temperatursensoren
Sensorbereiche 85 und 86 der Drucksensoren
Drucksensoren 85' und 86'
Waage 87
Temperatursensor 88
Temperatursensoren T1 bis T3
Temperatursensoren T4 und T5
Steuerung 90
Bilanzierung 95
Fluidwege 100
Hartteil 101
flexible Folie 102
Kassette 110
Schublade 111
Konnektorenaufnahme 112
Touchscreen 120
Anzeigeelemente 121 und 122
Kartenleser 125
Ankoppelfläche 130
Tür 140
Pumpaktoren 151 und 152
Interface-Rechner 150.
Aktionsrechner 160
Schutzrechner 170
Aktoren und Sensoren 180
Barcodeleser 190
Patientenkartenleser 200
Ein- und Ausgabeeinheit 210
Modem 220
Diagnosesystem 230

## Patentansprüche

1. Dialysemaschine (40), wobei es sich bei der Dialysemaschine um eine Peritonealdialysemaschine handelt, wobei mittels der Dialysemaschine (40) gemäß vorgegebenen und/oder eingebbaren Behandlungsparametern eine Peritonealdialysebehandlung durchführbar ist und wobei die Dialysemaschine (40) wenigstens ein Einstellmittel aufweist, mittels dessen wenigstens ein Behandlungsparameter während des Behandlungsbetriebes der Dialysemaschine (40) einstellbar ist, wobei die Dialysemaschine (40) ein Planungsmittel zur Planung eines Behandlungsverlaufes und ein Überwachungsmittel zur Überwachung des Behandlungsverlaufes aufweist, wobei mittels des Einstellmittels abgleichbar ist, ob ein tatsächlicher Behandlungsverlauf mit einem geplanten Behandlungsverlauf übereinstimmt oder abweicht,
mit zumindest einem der folgenden Merkmale A und B:
A) wenigstens die geplante Behandlungsdauer ist mittels des Planungsmittels vorgebbar und wenigstens die voraussichtliche Behandlungsdauer ist mittels des Überwachungsmittels anhand des bisherigen Behandlungsfortschritts errechenbar und/oder abschätzbar, wobei während der gesamten Behandlungsdauer mehrere Verweilzeiten (3) vorgesehen sind, deren Länge im Planungsmittel vorgebbar und/oder vorgegeben und eingespeichert sind, und wobei mittels des Einstellmittels anhand der Errechnung und/oder Abschätzung des Überwachungsmittels der voraussichtlichen Behandlungsdauer eine Korrektur wenigstens einer Verweilzeit (3) durchführbar ist, so dass die geplante Behandlungsdauer eingehalten werden kann; und
B) wenigstens ein geplantes Einlaufvolumen an Dialysat ist mittels des Planungsmittels vorgebbar und wenigstens das momentan verfügbare Volumen an Dialysat ist mittels des Überwachungsmittels errechenbar und/oder abschätzbar, wobei mittels des Einstellmittels anhand der Errechnung und/oder Abschätzung des Überwachungsmittels des momentan verfügbaren Volumens an Dialysat eine Korrektur wenigstens eines geplanten Einlaufvolumens und/oder aller noch geplanten Einlaufvolumina durchführbar ist, wobei die Dialysemaschine so ausgestaltet ist, dass vor Beginn der Behandlung ein zur Verfügung stehendes Mindestbeutelvolumen aller angeschlossenen Einlauflösungen maschinenseitig bekannt ist, indem diese identifiziert werden, wobei sich das Mindestbeutelvolumen aus einem nominalen Beutelfüllvolumen und einem garantierten Überfüllungsvolumen ergibt, die ebenfalls vor Beginn der Behandlung maschinenseitig bekannt sind,
wobei die Dialysemaschine hierdurch so ausgestaltet ist, dass:
das Überfüllungsvolumen für den Eigenverbrauch der Maschine verwendet wird; und
eine Anpassung erfolgt, wenn das Überfüllungsvolumen für den Eigenverbrauch der Maschine nicht ausreicht.

2. Dialysemaschine (40) nach Anspruch 1, insofern als umfassend das Merkmal A, **dadurch gekennzeichnet, dass** das Einstellmittel ein Begrenzungsmittel aufweist, mittels dessen ein prozentualer Mindestfaktor vorgebbar ist, der die maximal mögliche Korrektur der Verweilzeit (3) begrenzt, wobei der prozentuale Faktor vorzugsweise 10 % der geplanten Verweilzeit (3) beträgt.

3. Dialysemaschine (40) nach Anspruch 1 insofern als umfassend das Merkmal A oder nach Anspruch 2, **dadurch gekennzeichnet, dass** mittels des Einstellmittels die Zeitdifferenz von geplanter zu tatsächlicher Behandlungsdauer errechenbar ist und dass die errechnete Zeitdifferenz von der bislang vorgegebenen Verweilzeit (3) mittels des Einstellmittels abziehbar und eine dementsprechend um die errechnete Zeitdifferenz korrigierte Verweilzeit (3) einstellbar ist.

4. Dialysemaschine (40) nach Anspruch 1 insofern als umfassend das Merkmal B, **dadurch gekennzeichnet, dass** mittels des Planungsmittels alle noch geplanten Einlaufvolumina an Dialysat vorgebbar sind.

5. Dialysemaschine (40) nach Anspruch 1 insofern als umfassend das Merkmal B, **dadurch gekennzeichnet, dass** das Einstellmittel ein Begrenzungsmittel aufweist, mittels dessen ein prozentualer Mindestfaktor vorgebbar ist, der die maximal mögliche Korrektur des geplanten Einlaufvolumens begrenzt, wobei der prozentuale Faktor vorzugsweise 10 % des geplanten Einlaufvolumens beträgt.

6. Dialysemaschine (40) nach Anspruch 1 insofern als umfassend das Merkmal B oder nach Anspruch 5, **dadurch gekennzeichnet, dass** mittels des Einstellmittels die Volumendifferenz von geplantem zu tatsächlichem Einlaufvolumen bzw. von den geplanten zu den tatsächlichen Einlaufvolumina errechenbar ist und dass das errechnete Einlaufvolumen bzw. die errechneten Einlaufvolumina von dem bislang vorgegebenen Einlaufvolumen bzw. von den bislang vorgegebenen Einlaufvolumina mittels des Einstellmittels abziehbar und dementsprechend um die errechnete Volumendifferenz ein korrigiertes Einlaufvolumen bzw. korrigierte Einlaufvolumina einstellbar ist bzw. sind.

7. Dialysemaschine nach einem der vorangegangenen Ansprüche, wobei der wenigstens eine Behandlungsparameter mittels des Einstellmittels automatisch, dynamisch und während des Behandlungsbetriebes der Dialysemaschine einstellbar ist, wobei das Einstellmittel einen Zeitmanager und einen Volumenmanager aufweist.

## Claims

1. A dialysis machine (40), wherein the dialysis machine is a peritoneal dialysis machine, wherein a peritoneal dialysis treatment, can be carried out by means of the dialysis machine (40) in accordance with predefined and/or inputtable treatment parameters, and wherein the dialysis machine (40) has at least one setting means by means of which at least one treatment parameter can be set during the treatment operation of the dialysis machine (40), wherein the dialysis machine (40) has a planning means for planning a course of treatment and a monitoring means for monitoring the course of treatment, with it being able to be compared by means of the setting means whether an actual course of treatment corresponds to or differs from a planned course of treatment, having at least one of the following features A and B:
A)
at least the planned duration of treatment can be predefined by the planning means, and
at least the provisional treatment duration can be calculated and/or estimated by the monitoring means with reference to the previous treatment progress, wherein a plurality of dwell times (3) are provided during the total treatment duration whose lengths are predefinable and/or predefined and stored in the planning means and wherein a correction of at least one dwell time (3) can be carried out by means of the setting means with reference to the calculation and/or estimation of the monitoring means of the probable treatment duration so that the planned treatment duration can be adhered to;
and
B)
at least one planned instillation volume of dialysate can be predefined by means of the planning means, and at least the instantaneously available volume of dialysate can be calculated and/or estimated by means of the monitoring means,
wherein a correction of at least one planned instillation volume and/or of all still planned instillation volumes can be carried out by means of the setting means with reference to the calculation and/or estimation of the monitoring means of the instantaneously available volume of dialysate, wherein the dialysis machine is adapted such that an available minimum bag volume of all the connected instillation solutions is known at the machine side prior to the start of the treatment in that they are identified, with the minimum bag volume resulting from a nominal bag filling volume and from a guaranteed overfilling volume that are likewise known at the machine side prior to the start of the treatment,
wherein the dialysis machine is hereby adapted such that the overfilling volume is used for the machine's own consumption; and
an adaptation takes place when the overfilling volume is not sufficient for the machine's own consumption.

2. A dialysis machine (40) in accordance with claim 1, to the extent feature A is comprised, **characterized in that** the setting means has a limiting means by means of which a minimum percentage factor can be predefined which limits the maximum possible correction of the dwell time (3), with the percentage factor preferably amounting to 10% of the planned dwell time (3).

3. A dialysis machine (40) in accordance with claim 1, to the extent feature A is comprised, or in accordance with claim 2, **characterized in that** the time difference between the planned and actual treatment duration can be calculated by means of the setting means; and **in that** the calculated time difference can be deducted from the previously predefined dwell time (3) by means of the setting means and a dwell time (3) accordingly corrected by the calculated time difference can be set.

4. A dialysis machine (40) in accordance with claim 1, to the extent feature B is comprised, **characterized in that** all the still planned instillation volumes of dialysate can be predefined by means of the planning means.

5. A dialysis machine (40) in accordance with claim 1, to the extent feature B is comprised, **characterized in that** the setting means has a limiting means by means of which a minimum percentage factor can be predefined which limits the maximum possible correction of the planned instillation volume, with the percentage factor preferably amounting to 10% of the planned instillation volume.

6. A dialysis machine (40) in accordance with claim 1, to the extent feature B is comprised, or in accordance with claim 5, **characterized in that** the volume difference between the planned and actual instillation volume or between the planned and actual instillation volumes can be calculated by means of the setting means; and **in that** the calculated instillation volume or the calculated instillation volumes can be deducted from the previously predefined instillation volume or from the previously predefined instillation volumes by means of the setting means and an instillation volume or instillation volumes corrected by the calculated volume difference can accordingly be set.

7. A dialysis machine in accordance with one of the preceding claims, wherein the at least one treatment parameter can be set by the setting means automatically, dynamically, and during the treatment operation of the dialysis machine, with the setting means having a time manager and a volume manager.

## Revendications

1. Machine de dialyse (40), la machine de dialyse étant une machine de dialyse péritonéale, un traitement de dialyse péritonéale pouvant être effectué au moyen de la machine de dialyse (40) selon des paramètres de traitement prédéfinis et/ou pouvant être saisis et la machine de dialyse (40) comportant au moins un moyen de réglage, au moyen duquel au moins un paramètre de traitement peut être réglé pendant le fonctionnement en mode traitement de la machine de dialyse (40), la machine de dialyse (40) comportant un moyen de planification pour planifier un déroulement du traitement et un moyen de surveillance pour surveiller le déroulement du traitement, le moyen de réglage permettant de comparer si un déroulement réel du traitement concorde avec ou diffère d'un déroulement planifié du traitement, avec au moins une des caractéristiques A et B suivantes :
A) au moins la durée de traitement planifiée peut être prédéfinie au moyen du moyen de planification et
au moins la durée de traitement prévisionnelle peut être calculée et/ou estimée au moyen du moyen de surveillance en se fondant sur l'avancement actuel du traitement,
plusieurs temps de stase (3) étant prévus pendant toute la durée de traitement, dont la longueur peut être prédéfinie et/ou est prédéfinie et enregistrée dans le moyen de planification, et une correction au moins d'un temps de stase (3) pouvant être effectuée au moyen du moyen de réglage en se fondant sur le calcul et/ou l'estimation par le moyen de surveillance de la durée de traitement prévisionnelle, de telle sorte que la durée de traitement planifiée peut être respectée ; et
B)
au moins un volume d'entrée planifié de dialysat peut être prédéfini au moyen du moyen de planification et au moins le volume actuellement disponible de dialysat peut être calculé et/ou estimé au moyen du moyen de surveillance,
une correction au moins d'un volume d'entrée planifié et/ou de tous les volumes d'entrée encore planifiés pouvant être effectuée au moyen du moyen de réglage en se fondant sur le calcul et/ou l'estimation par le moyen de surveillance du volume actuellement disponible de dialysat, la machine de dialyse étant configurée de telle manière qu'un volume minimal de poche disponible de toutes les solutions d'entrée raccordées est connu côté machine avant le début du traitement, par le fait que celles-ci sont identifiées, le volume minimal de poche résultant d'un volume nominal de remplissage de poche et d'un volume garanti de surremplissage, qui sont également connus côté machine avant le début du traitement,
la machine de dialyse étant ainsi configurée de telle sorte que :
le volume de surremplissage est utilisé pour la consommation propre de la machine ; et
une adaptation est effectuée quand le volume de surremplissage ne suffit pas pour la consommation propre de la machine.

2. Machine de dialyse (40) selon la revendication 1, dans la mesure où elle comprend la caractéristique A, **caractérisée en ce que** le moyen de réglage comporte un moyen de limitation, au moyen duquel un facteur minimal en pourcentage peut être prédéfini, qui limite la correction maximale possible du temps de stase (3), le facteur en pourcentage étant de préférence de 10 % du temps de stase (3) planifié.

3. Machine de dialyse (40) selon la revendication 1, dans la mesure où elle comprend la caractéristique A ou selon la revendication 2, **caractérisée en ce que** la différence de temps entre la durée de traitement planifiée et réelle peut être calculée au moyen du moyen de réglage et **en ce que** la différence de temps calculée peut être retirée du temps de stase (3) prédéfini jusqu'ici au moyen du moyen de réglage et un temps de stase (3) corrigé en conséquence de la différence de temps calculée peut être réglé.

4. Machine de dialyse (40) selon la revendication 1, dans la mesure où elle comprend la caractéristique B, **caractérisée en ce que** tous les volumes d'entrée encore planifiés de dialysat peuvent être prédéfinis au moyen du moyen de planification.

5. Machine de dialyse (40) selon la revendication 1, dans la mesure où elle comprend la caractéristique B, **caractérisée en ce que** le moyen de réglage comporte un moyen de limitation, au moyen duquel un facteur minimal en pourcentage peut être prédéfini, qui limite la correction maximale possible du volume d'entrée planifié, le facteur en pourcentage étant de préférence de 10 % du volume d'entrée planifié.

6. Machine de dialyse (40) selon la revendication 1, dans la mesure où elle comprend la caractéristique B ou selon la revendication 5, **caractérisée en ce que** la différence de volume entre le volume d'entrée planifié et réel ou les volumes d'entrée planifiés et réels peut être calculée au moyen du moyen de réglage et **en ce que** le volume d'entrée calculé ou les volumes d'entrée calculés peut ou peuvent être retirés du volume d'entrée prédéfini jusqu'ici ou des volumes d'entrée prédéfinis jusqu'ici au moyen du moyen de réglage et un volume d'entrée corrigé ou des volumes d'entrée corrigés en conséquence de la différence de volume calculée peut ou peuvent être réglés.

7. Machine de dialyse selon l'une des revendications précédentes, dans laquelle l'au moins un paramètre de traitement peut être réglé au moyen du moyen de réglage automatiquement, dynamiquement et pendant le fonctionnement en mode traitement de la machine de dialyse, le moyen de réglage comportant un gestionnaire de temps et un gestionnaire de volume.
